# EUROPÄISCHE PATENTSCHRIFT

(11) **EP 1 327 682 B1**
(45) Veröffentlichungstag und Bekanntmachung des Hinweises auf die Patenterteilung: **13.05.2009**
(21) Anmeldenummer: 02000720.9
(22) Anmeldetag: 11.01.2002
(51) Int. Cl.: C12N 15/10, C12N 15/11, C12P 19/34

(54) **Verfahren zur Herstellung von DNA**
Method for producing DNA
Procédé de production de l'ADN

(43) Veröffentlichungstag der Anmeldung: 16.07.2003
(73) Patentinhaber: BioSpring Gesellschaft für Biotechnologie mbH, 60386 Frankfurt (DE)
(72) Erfinder: Aygün, Hüseyin, 60322 Frankfurt am Main (DE); Kircher, Markus, Dr., 60318 Frankfurt am Main (DE); Rosmus, Susann, Dr., 60318 Frankfurt am Main (DE); Wojczewski, Sylvia, Dr., 65812 Bad Soden (DE)
(74) Vertreter: Keller, Günter

(56) Entgegenhaltungen:
- EP-A- 0 585 660
- EP-A- 0 744 470
- WO-A-00/63437
- WO-A-01/57269
- WO-A-94/03636
- WO-A-96/15271
- JAYARAMAN K ET AL: "A PCR-mediated gene synthesis strategy involving the assembly of oligonucleotides representing only one of the strands" BIOTECHNIQUES, Bd. 3, Nr. 12, 1992, Seiten 392-398, XP001057259 ISSN: 0736-6205
- MEHTA DEEPA V ET AL: "Optimized gene synthesis, high level expression, isotopic enrichment, and refolding of human interleukin-5." PROTEIN EXPRESSION AND PURIFICATION, Bd. 11, Nr. 1, 1997, Seiten 86-94, XP002199111 ISSN: 1046-5928

## Beschreibung

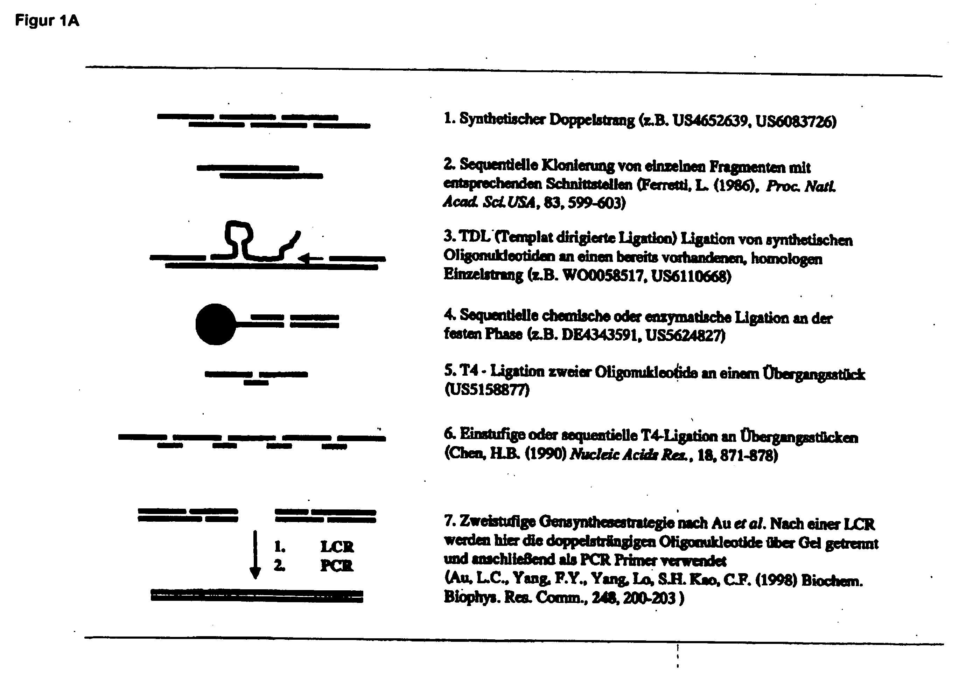

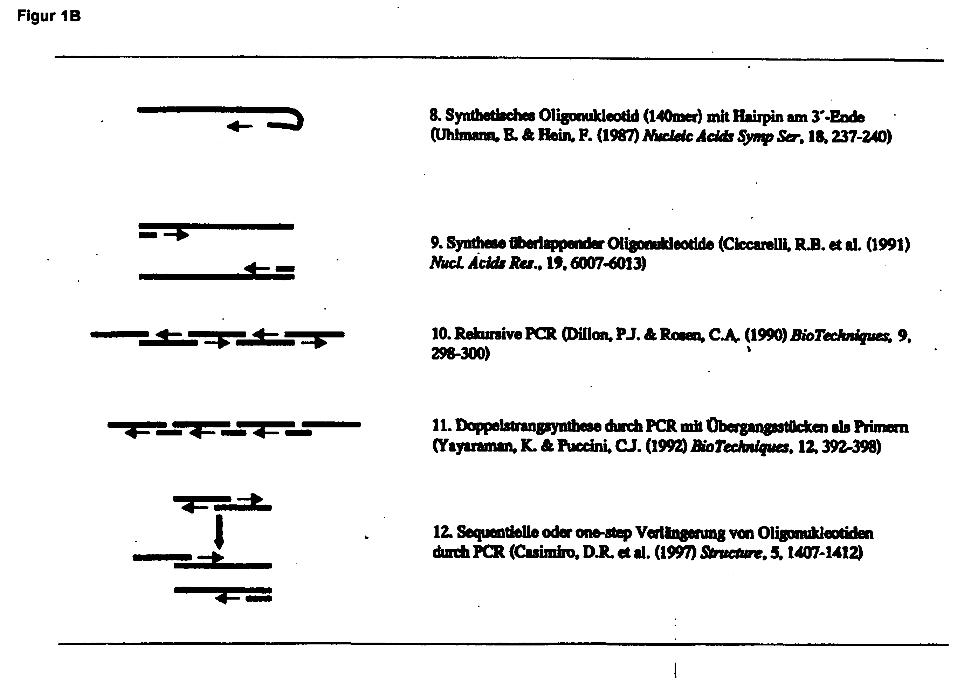

Die Erfindung betrifft ein Verfahren auf dem Gebiet der Nukleinsäuresynthese. Derzeit gibt es zahlreiche Verfahren zur Synthese von einzelsträngiger bzw. doppelsträngiger DNA (vgl. Figur 1A und 1B).

Die einfachste Art einer Einzelstrangsynthese besteht im chemischen Aufbau einzelsträngiger DNA. Entsprechend kann auch doppelsträngige DNA generiert werden, indem nach chemischer Synthese von Strang (+) bzw. Gegenstrang (-) eine Hybridisierung beider Stränge durchgeführt wird. Diese Technik stößt jedoch sehr schnell an ihre Grenzen. Mit den Standardverfahren der DNA-Synthese können selten Längen von über 150 Basen aufgebaut werden. Hinzu kommen Abbrüche und Verkürzungen, die sich nur über sehr aufwendige Reinigungsverfahren (Gelelektrophorese) wirkungsvoll von dem Hauptprodukt abtrennen lassen.

Die Vervollständigung einzelsträngiger DNA zu einem Doppelstrang lässt sich auch auf enzymatischem Weg realisieren. Hierbei können beispielsweise Außenprimer verwendet werden, die gezielt den Zwischenbereich in einer Polymerase-Kettenreaktion (PCR) amplifizieren. Bei einer anderen Technik werden längere Oligonukleotide an ihrem 3' Ende mit Hairpinmustern ausgestattet, die sich selbstkomplementär zusammenlagern und damit als "intramolekularer" Primer für eine enzymatische Verlängerung dienen (Uhlmann, 1987; siehe auch Figur 1B, Nr. 8).

Längere Oligonukleotide sind auch die Grundlage einer weiteren Technik, bei der vollständig überlappende Oligonukleotide, also ein Doppelstrang, mittels spezifischer Primer in einer PCR aufgefüllt werden (Ciccarelli, 1991; siehe auch Figur 1B, Nr. 9).

Die Verwendung der bisher beschriebenen Techniken wird jedoch durch die Länge der eingesetzten Oligonukleotide eingeschränkt.. Eine Erweiterung der Gensynthese auf größere Genabschnitte kann durch den Aufbau von sogenannten Genkassetten erreicht werden (US 4 652 639, US 6 083 726; siehe auch Figur 1A, Nr. 1 und 2). Solche Genkassetten bestehen aus kurzen, doppelsträngigen DNA Fragmenten, die entweder spezifische Überhänge von 3 bis 7 Basen (sticky end) oder auch glatte Enden (blunt end) mit 5' Phosphatgruppen tragen können. Überhänge haben dabei den Vorteil, dass durch eine definierte Auswahl solcher bei einer enzymatischen Ligation mehrere Fragmente gleichzeitig zu einem Gen kombiniert werden können. Der Aufbau dieser Kassetten erfolgt wiederum über Einzelstrangsynthese mit anschließender Hybridisierung von Strang (+) und Gegenstrang (-). Die 5' Phosphatgruppen werden vor Hybridisierung über Nukleotidkinasen an das Oligonukleotid angehängt. Durch die geringe Ligationseffizienz ist man bei der Verwendung einer solchen Strategie häufig auf eine Zwischenklonierung einzelner Genfragmente angewiesen (Ferretti, 1986). Zudem bereitet bei einer solchen Technik der Einsatz von degenerierten Oligonukleotiden, beispielsweise zum Aufbau von DNA Bibliotheken, große Schwierigkeiten.

Einfacher sind Reaktionen, die sequentielle Verlängerungstechniken auf PCR-Basis einsetzen (Ausubel, 1994; Jayaraman, 1991; Chang, 1993; Dillon, 1990: Jayaraman, 1992; Ye, 1992). Hierzu gehört beispielsweise eine Synthesetechnik, die 1997 von Casimiro beschrieben wurde (Casimiro, 1997; siehe auch Figur 1B, Nr. 12). In dieser Technik wird sukzessive doppelsträngige DNA generiert, indem lange einzelsträngige Oligonukleotide mit komplementären Enden über PCR amplifiziert werden und somit als Matrizen ("templates") für verlängernde PCR-Reaktionen dienen können. Ein wesentlicher Nachteil einer solchen Strategie besteht in der Akkumulation von Mutationen durch die Verwendung zu hoher Zyklenzahlen in der PCR. Bei der rekursiven Gensynthesestrategie (Dillon, 1990; Ausubel, 1994; Traub, 2001; siehe auch Figur 1B, Nr. 10) werden die zu synthetisierenden Gene als zueinander versetzt überlappende, Strang (+) und Gegenstrang (-) Oligonukleotide aufgebaut. In einer anschließenden PCR können die freien 3'-Enden solcher Oligonukleotide als Primer zur Synthese des komplementären Strangabschnittes eingesetzt werden. Da sich nach jeder Verlängerung wieder neue Anlagerungsmöglichkeiten für flankierende Sequenzen ergeben, lassen sich so Zyklus-für-Zyklus vollständige Gene synthetisieren. In einer etwas abgewandelten Form, kommt diese Strategie auch bei einem anderen Verfahren zum Einsatz. Hier verzichtet man auf die Verwendung besonders langer Oligonukleotide zur Verlängerung des Gens und arbeitet stattdessen mit kleineren Übergangsstücken, die die einzelnen Genfragmente miteinander verbinden. Diese Übergangsstücke fungieren gleichzeitig in der PCR als Primer, die den komplementären Gegenstrang entsprechend aufbauen (Yayaraman, 1992; siehe auch Figur 1A, Nr. 11). Genau wie bei Casimiro (Casimiro, 1997), besteht auch hier ein wesentlicher Nachteil in der Akkumulation von Mutationen durch die Verwendung zu hoher Zyklenzahlen in der PCR. Weiterhin verhindern die einzelnen doppelsträngigen Fragmente eine effiziente Amplifikation des Vollängenproduktes, da sie bedingt durch Ihre Länge bei wesentlich höheren Temperaturen mit der Matrize hybridisieren als die PCR-Außenprimer.

In wiederum anderen Gensynthesestrategien steht der Einsatz von Ligasen im Vordergrund (Sproat, 1985; Ferretti, 1986; Hostomsky, 1987; Wosnick, 1989; Climie, 1990; Oprian, 1991). Bei der TDL-Technologie ("template directed ligation") werden Oligonukleotide mit 5'-Phosphatgruppen an einen bereits vorhandenen Einzelstrang hybridisiert und anschließend enzymatisch zu Oligonukleotidpolymeren verknüpft (WO 0058517, US 6 110 668; siehe auch Figur 1A, Nr. 3). Ein solcher Gegenstrang lässt sich entweder durch vorherige Exonukleasebehandlung eines entsprechenden Wildtyptemplates oder aus einer asymmetrischen PCR zur Verfügung stellen. Diese Gensynthesestrategie ist jedoch auf die Erzeugung bzw. Reproduktion von homologen Genen begrenzt. Mit Hilfe der T4-DNA Ligase lassen sich auch Paare von Oligonukleotiden mit Hilfe deutlich kürzerer Übergangsstücke miteinander verknüpfen (US 5 158 877; siehe auch Figur 1A, Nr. 5). Eine solche Ligation setzt auch hier eine Phosphatgruppe am 5'-Ende des stromabwärts (zum 3'-Ende hin) gelegenen Oligonukleotides vorraus. Eine Variante dieses Verfahrens geht von einer deutlich höheren Anzahl einzelsträngiger Oligonukleotide aus, die schließlich in einem gemeinsamen Ligationsschritt durch die T4-DNA Ligase miteinander verknüpft werden (Chen, 1990; Figur 1A, Nr. 6). Eine solche Ligation lässt sich entweder in einem Schritt (all-in-one) oder aber auch sequentiell durchführen. Ein Nachteil bei den Einzelstrangtechniken ist das Fehlen eines Gegenstranges zur entsprechenden Klonierung in Vektoren. Chen et al. konnten jedoch zeigen, daß die direkte Klonierung von Einzelsträngen in zuvor geöffnete Vektoren durchaus möglich ist. Die Verwendung von T4-DNA Ligasen schränkt zudem die Ligationsbedingungen auf Temperaturen um 37°C ein. Dadurch können Sekundärstrukturen, die bei langen, einzelsträngigen Oligonukleotiden häufig entstehen, die Ligation nachteilig beeinflussen.

Andere Gensynthesestrategien kombinieren die Vorteile aus Ligase- bzw. Polymerasebasierenden Teilschritten (Au, 1998; Chalmers, 2001; siehe auch Figur 1A, Nr. 7). Die von Au et al. vorgestellte Synthesestrategie (Au, 1998) geht von zueinander komplementären Oligonukleotiden aus (um 40 Nukleotide), die zunächst mit Hilfe thermostabiler Ligasen (Pfu-DNA Ligase) in einer Ligase-Kettenreaktion (LCR) zu doppelsträngigen Teilfragmenten kombiniert werden. Diese Fragmente werden anschließend isoliert und über PCR neu miteinander kombiniert.

Von diesen sich in Lösung abspielenden Reaktionen weichen Techniken ab, die den Aufbau von Genen auf fester Phase (z.B. auf Beads) als Grundlage haben (US6083726, W09517413; siehe auch Figur 1A, Nr. 4). Eine Verknüpfung mit einer solchen Phase kann entweder über die terminale Modifikation von DNA mit hochaffinen Bindungsmolekülen (Biotin, Digoxigenin) oder mit funktionalen Gruppen (NH2, COOH, SH) erreicht werden. Diese Synthesestrategien haben den großen Vorteil, dass Fragmente, die bei einer Ligation nicht eingebaut werden, bei den anschließenden Waschschritten entfernt werden können. Der sequentielle Aufbau größerer Gene kann, basierend auf einer solchen Festphasenstrategie, entweder chemisch (z.B. über 5'lod- bzw. 3'Thiophosphatmodifizierte Oligonukleotide) oder enzymatisch z.B. durch repetitiven Verdau mit Alw261 (US 6 083 726) erfolgen. Beim enzymatischen Aufbau wird die T4-DNA Ligase, zur blunt end bzw. sticky end Ligation der doppelsträngigen Genfragmente oder die T4-RNA-Ligase zur Ligation der einzelsträngigen Oligonukleotide verwendet (WO 9517413).

Eine Aufgabe der vorliegenden Erfindung ist es ein vorteilhaftes Verfahren zur Herstellung von DNA zur Verfügung zu stellen.

Überraschenderweise wurde gefunden, daß durch eine Exonukleasereaktion nach Ligation an scharnierartigen Übergangsstücken das gewünschte Produkt oder Zwischenprodukt besonders angereichert und/oder selektiert werden kann..

Die Erfindung betrifft daher ein Verfahren zur Herstellung von DNA, das eine matrizenabhängige Ligation ("template directed ligation") an Übergangsstücken und eine nachfolgende Exonukleasereaktion umfaßt.

Ein erster Aspekt der Erfindung ist ein Verfahren zur Herstellung von DNA, das umfasst, daß man
a) n einzelsträngige Basis-DNA-Oligonukleotide bereitstellt, die unmittelbar aufeinanderfolgende Teile der Nukleotidsequenz der herzustellenden DNA sind, wobei
   i) das zweite bis n-te Basis-DNA-Oligonukleotid am 5'-Ende phosphoryliert ist und
   ii) n 3 bis 100 ist ;
b) wenigstens (n-1) einzelsträngige Scharnier-DNA-Oligonukleotide bereitstellt, wobei für die Scharnier-DNA-Oligonukleotide gilt, daß der 3'-terminale Bereich eines Scharnier-DNA-Oligonukleotids wenigstens teilweise komplementär zum 3'-terminalen Bereich eines Basis-DNA-Oligonukleotids ist, und der 5'-terminale Bereich desselben Scharnier-DNA-Oligonukleotids wenigstens teilweise komplementär zum 5'-terminalen Bereich des unmittelbar darauffolgenden Basis-DNA-Oligonukleotids ist, so daß im Falle der Hybridisierung eines Scharnier-DNA-Oligonukleotids mit 2 unmittelbar aufeinanderfolgenden Basis-DNA-Oligonukleotiden ein im Bereich des Scharnier-DNA-Oligonukleotids doppelsträngiges DNA-Hybrid entsteht;
c) die Basis-DNA-Oligonukleotide mit den Scharnier-DNA-Oligonukleotiden in Kontakt bringt;
d) das aus Schritt c) resultierende DNA-Hybrid einer Ligationsreaktion unterwirft.
e) das Reaktionsprodukt aus Schritt d) einer Exonukleasereaktion unterwirft, wobei der durch ligierte Basis-DNA-Oligonukleotide gebildete DNA-Strang des Reaktionsprodukts aus Schritt d) wenigstens zwei Cap-Strukturen umfasst, wobei das Reaktionsprodukt der Exonukleasebehandlung einzelsträngige DNA mit Cap-Strukturen an ihren Enden ist, und wobei eine Cap-Struktur eine Struktur ist, die einem Ende einer linearen Nukleinsäure Resistenz gegen eine Exonuklease verleiht.

In einem ersten Schritt des Verfahrens werden n einzelsträngige Basis-DNA-Oligonukleotide bereitstellt, die unmittelbar aufeinanderfolgende Teile der Nukleotidsequenz der herzustellenden DNA sind, wobei n 3 bis 100, bevorzugter 5 bis 50, am bevorzugtesten 7 bis 25 ist.

Der Ausdruck "Oligonukleotid" in dieser Anmeldung bedeutet keine besondere Einschränkung bezüglich der Länge des Oligonukleotids. Die Basis-DNA-Oligonukleotide haben üblicherweise eine Länge von 45 bis 1000 Nukleotiden, bevorzugt von 50 bis 500, bevorzugter von 75 bis 300, am bevorzugtesten von 100 bis 150 Nukleotiden. Die Basis-DNA-Oligonukleotide können auf verschiedene Weisen hergestellt werden. Üblich ist es aber, zur Herstellung die Phosphoramidit-Methode zur Synthese von Oligonukleotiden einzusetzen. Einzelheiten dieser Synthesemethode und zur Durchführung geeignete Vorrichtungen sind dem Fachmann bekannt und können beispielsweise Beaucage, S.L. & lyer, R.P. (1993) Tetrahedron, 49 (28), 6123-6194; Caruthers, M.H. et al. (1987) Methods in Enzymol., 154, 287-313; Beaucage, S.L. & Caruthers, M.H. (1981) Tetrahedron Lett. 22 (20), 1859-1862 entnommen werden.

Das "erste" Basis-DNA-Oligonukleotid ist das in der herzustellenden DNA am meisten 5' gelegene Basis-DNA-Oligonukleotid, bezogen auf den Strang, dessen Sequenz der Sequenz des Basis-DNA-Oligonukleotids entspricht. Die Sequenz des "zweiten" Basis-DNA-Oligonukleotids schließt sich unmittelbar an das 3'-Ende des "ersten" Basis-DNA-Oligonukleotids an. Das "n-te" oder "letzte" Basis-DNA-Oligonukleotid ist das in der herzustellenden DNA am meisten 3' gelegene Basis-DNA-Oligonukleotid, bezogen auf den Strang, dessen Sequenz der Sequenz des Basis-DNA-Oligonukleotids entspricht.

Die Basis-DNA-Oligonukleotide mit Ausnahme des ersten Basis-DNA-Oligonukleotids sind am 5'-Ende phosphoryliert. Dies ist für die spätere Ligation erforderlich. Die Phosphorylierung kann nach der Synthese der Oligonukleotide in einer separaten Reaktion erfolgen. Bevorzugt ist es aber, die Phosphorylierung unmittelbar am Ende der Oligonukleotidsynthese im DNA-Synthesizer durchzuführen. Die Durchführung dieser Methode ist dem Fachmann bekannt.

In einem weiteren Schritt des Verfahrens werden wenigstens (n-1) einzelsträngige Scharnier-DNA-Oligonukleotide bereitstellt. Die Scharnier-DNA-Oligonukleotide haben in der Regel eine Länge von 8 bis 300 Nukleotiden, bevorzugt von 10 bis 100, bevorzugter von 16 bis 70 Nukleotiden, am bevorzugtesten von 20 bis 40 Nukleotiden. Auch die Scharnier-DNA-Oligonukleotide werden vorzugsweise durch die Phosphoramidit-Methode hergestellt.

Scharnier-DNA-Oligonukleotide sind Oligonukleotide, die durch Hybridisierung mit 2 unmittelbar aufeinanderfolgenden Basis-DNA-Oligonukleotiden zu einem DNA-Hybrid führen können, das einen doppelsträngigen und zwei einzelsträngige Bereiche aufweist. Das Scharnier-DNA-Oligonukleotid erfüllt damit die Funktion einer Ligationsmatrize, da es zwei unmittelbar aufeinanderfolgende Basis-DNA-Oligonukleotide räumlich zueinander führt, so daß unter geeigneten Bedingungen eine Ligation erfolgen kann. Der 3'-terminale Bereich eines Scharnier-DNA-Oligonukleotids ist also wenigstens teilweise komplementär zum 3'-terminalen Bereich eines bestimmten Basis-DNA-Oligonukleotids, und der 5'-terminale Bereich desselben Scharnier-DNA-Oligonukleotids wenigstens teilweise komplementär zum 5'-terminalen Bereich des unmittelbar darauffolgenden Basis-DNA-Oligonukleotids, so daß im Falle der Hybridisierung eines Scharnier-DNA-Oligonukleotids mit,2 unmittelbar aufeinanderfolgenden Basis-DNA-Oligonukleotiden ein im Bereich des Scharnier-Oligonukleotids doppelsträngiges DNA-Hybrid entsteht.

Die Komplementarität muß nicht 100% sein, sie muß aber ausreichen, um eine Hybridisierung unter geeigeten Bedingungen zu gewährleisten. Bevorzugt ist eine Identität von wenigstens 95 %. In einer besonderen Ausführungsform ist die Komplementarität 100 %.

Die Länge des mit einem bestimmten Basis-DNA-Oligonukleotid hybridisierenden Bereichs eines Scharnier-DNA-Oligonukleotids hängt in erster Linie von der Gesamtlänge des Scharnier-DNA-Oligonukleotids ab. Üblicherweise kann die 5'-terminale Hälfte eines Scharnier-DNA-Oligonukleotids mit dem einen Basis-DNA-Oligonukleotide hybridisieren, die 3'-terminale Hälfte des Scharnier-DNA-Oligonukleotids mit einem anderen. Es kann aber durchaus eine Abweichung von dieser hälftigen Aufteilung gegeben sein.

In einer besonderen Ausführungsform sind die Scharnier-DNA-Oligonukleotide so modifiziert, daß sie am 3'-Ende nicht enzymatisch verlängert werden können, z. B. durch DNA-Polymerasen.

In einem dritten Schritt des Verfahrens werden die Basis-DNA-Oligonukleotide mit den Scharnier-DNA-Oligonukleotiden in Kontakt gebracht. Dies erfolgt unter solchen Bedingungen, daß Hybridisierungen zwischen dem oder den Scharnier-DNA-Oligonukleotid(en) und den Basis-DNA-Oligonukleotiden stattfinden können.

In einem weiteren Schritt wird das aus dem vorhergehenden Schritt resultierende DNA-Hybrid einer Ligationsreaktion unterworfen. Dieser Schritt kann auch im wesentlichen gleichzeitig mit dem dritten Schritt zusammen vorgenommen werden, d. h. die verschiedenen Oligonukleotide werden einfach zusammen mit den Ligationsreagenzien gemischt und unter solchen Bedingungen inkubiert, daß eine Ligation stattfinden kann.

Es können verschiedene Enzyme mit Ligase-Aktivität zur Ligation eingesetzt werden, beispielsweise T4-DNA-Ligase, die in einem Temperaturbereich von 16°C bis 37°C die höchste Aktivität aufweist. Es hat sich aber als besonders vorteilhaft herausgestellt, eine thermostabile Ligase zu verwenden. Dadurch können selbst bei langen Basis-DNA-Oligonukleotiden (mit einer Länge >150 Nukleotide) bei erhöhter Temperatur noch gute Ligationsausbeuten erhalten werden. Bevorzugte Enzyme sind *Taq* DNA-Ligase und *Pfu* DNA-Ligase.

Wesentlich für das erfindungsgemäße Verfahren ist, daß das Reaktionsprodukt der Ligationsreaktion in einem fünften Schritt einer Exonukleasereaktion unterworfen wird. "Exonuklease" im Sinne dieser Anmeldung ist ein Enzym, das Nukleotide sequenziell von freien Enden eines linearen Nukleinsäuresubstrats abspaltet. Im Gegensatz dazu spaltet eine "Endonuklease" das Nukleinsäuresubstrat an internen Stellen der Nukleotidsequenz.

Diese Reaktion kann sich unmittelbar an die Ligation anschließen, es ist aber auch denkbar, daß zwischen Ligation und Exonuklease-Behandlung Zwischenschritte erfolgen. Das Reaktionsprodukt der Ligation kann nach der Ligation isoliert oder angereichert werden, beispielsweise durch Fällung der DNA. Es ist aber auch denkbar, daß das Reaktionsgemisch im wesentlichen unverändert der Exonuklease-Behandlung unterworfen wird.

Zur Exonuklease-Behandlung werden Enzyme mit Exonuklease-Aktivität eingesetzt. Möglich sind beispielsweise Exonuklease VII, allgemein Exonukleasen, bevorzugt Exonuklease VII, jedoch auch Exonuklease I, Exonuklease III und Exonuklease V als auch DNase I sowie Mischungen der soeben beschriebenen Hydrolasen.

Der durch ligierte Basis-DNA-Oligonukleotide gebildete DNA-Strang des Reaktionsprodukts enthält wenigstens 2 Cap-Strukturen. Eine "Cap-Struktur" im Sinne dieser Anmeldung ist eine Struktur, die einem Ende einer linearen Nukleinsäure Resistenz gegen eine Exonuklease verleiht. Dadurch ist die zu synthetisierende gewünschte DNA-Sequenz vor Nukleaseabbau geschützt. Eine erste Cap-Struktur liegt im 5'-terminalen Bereich der zu synthetisierenden DNA-Sequenz, eine weitere Cap-Struktur liegt im 3'-terminalen Bereich der zu synthetisierenden DNA-Sequenz.

Die Cap-Struktur kann, muß aber nicht am unmittelbaren 5'-bzw. 3'-Ende des durch ligierte Basis-DNA-Oligonukleotide gebildeten DNA-Strangs des Reaktionsprodukts liegen. Die Erfindung umfaßt auch den Fall, daß ein oder zwei Enden dieses Stranges Nukleotide aufweisen, die nicht gegen Exonuklease-Abbau geschützt sind. Wesentlich ist, daß die gewünschte DNA-Sequenz durch Cap-Strukturen geschützt ist. Es ist also auch der Fall umfaßt, daß durch Basis-DNA-Oligoukleotide an den Enden des entstehenden DNA-Strangs Nukleotide eingeführt werden, die nicht in der gewünschten DNA-Sequenz enthalten sein müssen. Derartige Nukleotide müssen nicht gegen Nukleaseabbau geschützt sein.

Dem Fachmann sind verschiedene Cap-Strukturen bekannt. Beispiele dafür sind Thioatbindungen zwischen einzelnen Nukleotiden, 2'OMethyl-RNA, modifizierte Basen, DNA-Sequenzen mit Schleifenstruktur(en) und/oder RNA-Sequenzen mit Schleifenstruktur(en). Basenmodifikationen, die Schutz gegen Exonuklease-Abbau verleihen, sind C-5 Propinyl bzw. C-5 Methyl modifizierte Basen, 2-Amino-2'-deoxy Adenin, N-4-Ethyl-2'-deoxy Cytidin, 2'-deoxy Inosin, 2'-deoxy Uridin sowie die unnatürlichen Basen Nebularin, Nitropyrrol und 5-Nitroindole.

Es gibt auch weitere 3' und 5' Modifikationen, die vor Nukleaseabbau schützen, wie primäre, sekundäre oder tertiäre Amine, die ebenso wie Hydroxyl- und Thiol-Gruppen über aliphatische oder durch Sauerstoff "O", Schwefel "S" bzw. Stickstoff "RR'R"N" modifiziertaliphatische Linker an terminalen Phosphatgruppen (3' bzw. 5' Phosphat) hängen, verzweigte und unverzweigte Ethylenglykole, genauso wie Glycerin Derivate. Eingesetzt werden können auch endständige Markierungen wie Biotin, Dinitrophenol, und Digoxigenin sowie alle handelsüblichen Farbstoffe, die unmittelbar als Phosphoramidite bzw. mittelbar als Aktivester erhältlich sind.

In der Regel wird eine erste Cap-Struktur durch das erste Basis-DNA-Oligonukleotid eingeführt, eine weitere Cap-Struktur durch das n-te-Basis-DNA-Oligonukleotid. Theoretisch wäre es auch denkbar, daß weiter innen liegende Basis-DNA-Oligonukleotide eine Cap-Struktur umfassen, dies ist aber nicht bevorzugt.

Das Reaktionsprodukt der Exonukleasebehandlung ist einzelsträngige DNA mit Cap-Strukturen an ihren Enden. Gemäß einer besonderen Ausführungsform der Erfindung kann diese einzelsträngige DNA durch PCR in doppelsträngige DNA überführt und vermehrt werden. Dazu werden vorzugsweise Primer verwendet, deren Zielsequenzen im 5'-terminalen Bereich bzw. im 3'-terminalen Bereich der gewünschten DNA-Sequenz liegen. Die Zielsequenzen liegen üblicherweise im Bereich des ersten bzw. letzten Basis-DNA-Oligonukleotids. Durch die Primer können auch Restriktionsschnittstellen an den Endbereichen der doppelsträngigen DNA eingeführt werden, die Primer enthalten dann eine Erkennungssequenz für eine oder mehrere Restriktionsendonukleasen. Das so hergestellte doppelsträngige DNA-Produkt kann durch Restriktionsenzyme entsprechend verdaut werden und beispielsweise in ein Plasmid oder einen Vektor kloniert werden. Die DNA kann dann in eine Zelle eingeführt werden. Dadurch kann die so hergestellte DNA beispielsweise in Bakterien vermehrt werden. Derartige Techniken sind dem Fachmann bekannt. Die DNA kann auch in eukaryontische Zellen, z. B. Säugerzellen eingeführt werden, um gewünschte Polypeptide zu exprimieren.

In einer besonderen Ausführungsform enthalten ein oder mehrere Basis-DNA-Oligonukleotide und/oder Scharnier-DNA-Oligonukleotide randomisierte Nukleotide. Dadurch kann DNA hergestellt werden, die an bestimmten Stellen Variationen aufweist. Der Einbau solcher Variationen in eine Sequenz erfolgt bereits während der Oligonukleotidsynthese. Durch Verwendung von DNA-Phosphoramiditmischungen, die anstatt der einzelnen Phosphoramidite sämtliche Basen (dA, dC, dG und dT) in einem bestimmten Verhältnis enthalten (N-Mischungen), gelangt man zu partiell oder vollständig randomisierten Oligonukleotiden. Diese Oligonukleotide können über das hier beschriebene Verfahren zu kompletten Genen vervollständigt werden und liefern, eingebaut in die entsprechenden Vektoren, die gewünschten Protein- oder Peptidbibliotheken. Solche Bibliotheken sind die Grundlage für die Suche nach bestimmten, neuen Eigenschaftsmustern. Bei Zumischung einer N-Mischung zu den einzelnen Monomeren (XN-Mischungen) besteht darüber hinaus die Möglichkeit, den Grad der Randomisierung einzuschränken. Hierdurch wird gewährleistet, daß die Variationsbreite innerhalb einer Protein- bzw. Peptidbibliothek im Verhältnis zum Ausgangsgen klein bleibt. Diese Strategie verhindert, daß entstandene positive Mutationen durch Überlagerung mit weiteren Mutationen in der Protein- oder Peptidbibliothek unterdrückt werden oder verlorengehen.

Die Erfindung betrifft außerdem ein DNA-Hybrid, das einen DNA-Einzelstrang einen oder mehrere damit hybridisierende Scharnier-DNA-Oligonukleotide sowie wenigstens zwei Cap-Strukturen umfaßt.

Die Erfindung betrifft auch ein Kit, das geeignet ist zur Durchführung des Verfahrens. Das erfindungsgemäße Kit enthält ein erstes Basis-DNA-Oligonukleotid, das eine Cap-Struktur umfaßt, ein weiteres Basis-DNA-Oligonukleotid, das eine Cap-Struktur umfaßt, ein Enzym mit Ligaseaktivität und ein Enzym mit Exonukleaseaktivität. Das Kit kann außerdem Reagenzien enthalten, die zur Durchführung eingesetzt werden können, z. B. konzentrierte Pufferlösungen.

Das Kit kann darüberhinaus Mittel zur Durchführung einer PCR enthalten. Derartige Mittel können Primer und eine thermostabile DNA-Polymerase sein. Die Primer enthalten vorzugsweise eine oder mehrere Erkennungssequenzen für eine oder mehrere Restriktionsendonukleasen.

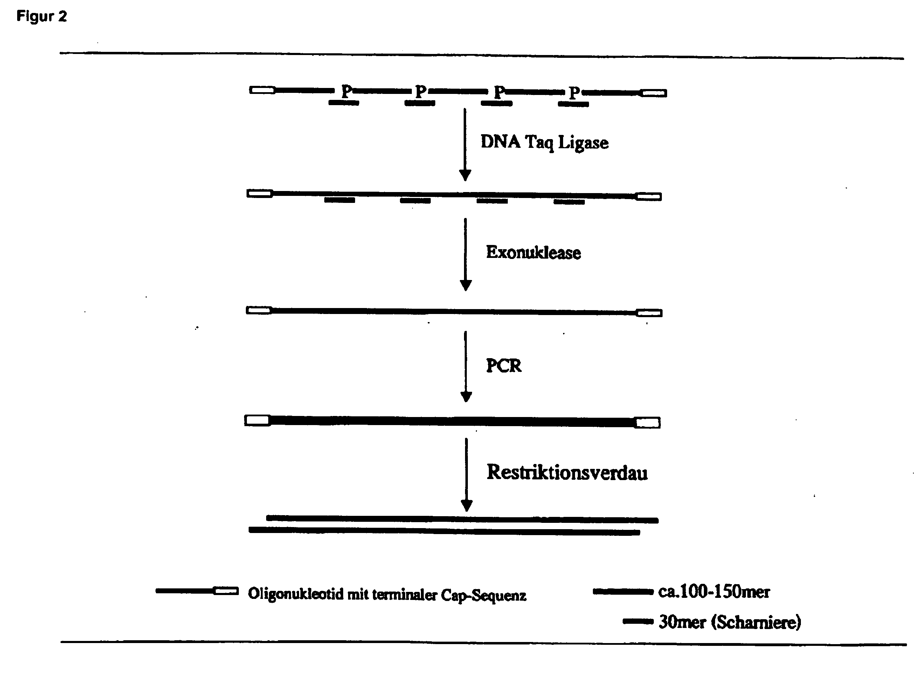

Das vorliegende Verfahren zur chemoenzymatischen Totalsynthese von Genen (vgl. Figur 2) zeichnet sich durch zahlreiche Vorteile gegenüber herkömmlichen \/erfahren aus:

Es erlaubt die totalsynthetische Konstruktion von Genen auf Basis besonders langer Basis-DNA-Oligonukleotide (45-1000 Basen). Eine Gegenstrangsynthese entfällt, was den Zeitaufwand und die Kosten zum Aufbau von Genen oder Genklustern deutlich reduziert. Im Vergleich zu anderen Gensynthesestrategien kommt man ohne die zeitaufwendige Zwischenklonierung von Genfragmenten aus. Zudem erlaubt der Aufbau lediglich eines Stranges die Einführung von Mutationen auf DNA-synthetischer Ebene. Mutagenisierte oder randomisierte Abschnitte können so an beliebiger Stelle des Gens generiert und bei der Gegenstrangsynthese (PCR) vervollständigt werden. Schwierigkeiten bei der Hybridisierung randomisierter Sequenzen entfallen hierdurch.

Eine Besonderheit des erfindungsgemäßen Verfahrens besteht in der Verwendung von Cap-Strukturen, insbesondere von 5' und 3' Überhängen, zur *in vitro* Selektion von Ligationsprodukten. Solche Cap-Strukturen bestehen aus 3' bzw. 5' Nukleaseresistenzen, die durch Polymerasen oder Enzyme mit Nukleaseaktivität (5' → 3' sowie 3' → 5') nicht verkürzt werden können. Das nach der Ligation aufgebaute Vollängenprodukt ist an beiden Enden vor Nukleaseabbau geschützt, alle kürzeren Zwischenprodukte oder eingesetzte Oligonukleotide, einschließlich der endständigen Oligonukleotide jedoch nicht. Dadurch wird das nach Nukleasebehandlung an beiden Enden geschützte Vollängenprodukt im Reaktionsansatz selektiert bzw. stark angereichert. Die sich üblicherweise anschließende PCR liefert das gewünschte doppelsträngige Genprodukt.

Die Synthese- und Aufreinigungsprotokolle können so modifiziert werden, dass besonders Die Synthese- und Aufreinigungsprotokolle können so modifiziert werden, dass besonders lange Oligonukleotide in hoher Qualität und Exaktheit erhalten werden können. Zudem erlaubt der Einsatz eines speziellen Phosphorylierungsreagenzes die Abtrennung ausschließlich terminal modifizierter Basis-DNA-Oligonukleotide, was die oligonukleotidspezifische Ligation (OSL) sehr effizient gestaltet.

Bereits beim Aufbau der Oligonukleotide können Faktoren, wie beispielsweise die Codon Usage auf den jeweiligen Wirt optimal angepasst werden, was die Expression heterologer Proteine teilweise erst ermöglicht.

Der Zusammenbau des Zielgens oder -clusters erfolgt enzymatisch mit Hilfe von kurzen komplementären Oligonukleotiden (Scharniere), die als Ligationsmatritzen fungieren. Dadurch entfällt die Notwendigkeit eines kompletten Gegenstranges zur spezifischen Ligation der Basis-DNA-Oligonukleotide Eine unerwünschte Einflussnahme dieser Scharniere in der nachfolgenden PCR kann durch die Verwendung von 3'Phosphatgruppen, die sich enzymatisch nicht verlängern lassen, unterbunden werden.

Für die OSL können neben den üblichen Ligasen wie z.B. die T4 DNA Ligase (16°C bis 37°C) auch thermostabile Ligasen wie z.B. *Taq* oder *Pfu* DNA Ligase (37°C-80°C) eingesetzt werden. Dadurch gelingt es häufig optimale Ligationsbedingungen ausfindig zu machen.

Die Totalsynthese von Zielgenen mit Hilfe des hier vorgestellten Verfahrens ermöglicht neue Möglichkeiten beim Aufbau von Genbibliotheken:
i) Beispielsweise können bestimmte Aminosäuren oder Sequenzabschnitte auf DNA-synthetischer Ebene vollständig randomisiert und somit in das Gen eingebracht werden.
ii) Andernfalls gelingt es mit Hilfe dieser Technologie auch "eingeschränkt-randomisierte" Sequenzen zu generieren, die beispielsweise lediglich hydrophobe Aminosäuren zulassen, im Gen zulassen (z.B. NTN).

Figur 1A und 1B zeigen schematisch die Prinzipien verschiedener Verfahren zur Herstellung von DNA (siehe auch oben).

Figur 2 zeigt schematisch eine bestimmte Ausführungsform des erfindungsgemäßen Verfahrens. Es werden fünf Basis-DNA-Oligonukleotide bereitgestellt, von denen das erste und fünfte jeweils eine Cap-Struktur enthalten. Die Basis-DNA-Oligonukleotide zwei bis fünf sind 5'-terminal phosphoryliert. Als Ligationsmatrizen fungieren vier Scharnier-DNA-Oligonukleotide, die unterhalb der Übergangsstellen der Basis-DNA-Oligonukleotide gezeigt sind. Durch die Ligation werden die Basis-DNA-Oligonukleotide zu einem Einzelstrang verbunden, an den noch die Scharnier-DNA-Oligonukleotide hybridisiert sind. Diese werden aber durch die Exonuklease abgebaut, während der ligierte Einzelstrang durch die Cap-Strukturen geschützt ist. Der Einzelstrang wird durch PCR in doppelsträngige DNA überführt. Mit der PCR wurden Schnittstellen eingeführt, so daß ein Restriktionsverdau erfolgen kann.

Figur 3 zeigt das in Beispiel 1 hergestellte Xylanasegen nach Ligation durch Taq-Ligase, Exonuklease-Behandlung und PCR-Amplifikation. Links ist ein 100bp Marker (New England Biolabs) aufgetragen, rechts 10µl des Amplifikationsansatzes auf einem 2%igen Agarosegel in 1xTBE.

Figuren 4A und 4B zeigen die durch DNA-Sequenzierung ermittelte Nukleotidsequenz der in Beispiel 1 hergestellten DNA nach Klonierung in den Vektor pET23a. Die ermittelte Sequenz des Xylanasegens stimmt mit der gewünschten Sequenz überein.

Figur 5 zeigt die in Beispiel 2 hergestellte Chymotrypsinogen A-DNA nach Ligation durch Taq-Ligase, Exonuklease-Behandlung und PCR-Amplifikation. Links ist ein 100bp Marker (New England Biolabs) aufgetragen, rechts 10µl des Amplifikationsansatzes auf einem 1.5%igen Agarosegel in 1xTBE.

Figuren 6A und 6B zeigen die durch DNA-Sequenzierung ermittelte Nukleotidsequenz der in Beispiel 2 hergestellten DNA nach Klonierung in den Vektor pET23a. Die ermittelte Sequenz des Gens für Chymotrypsinogen A stimmt mit der gewünschten Sequenz überein.

Die nachfolgenden Beispiele erläutern die Erfindung näher.

### Beispiel 1: Synthese des Xylanase Gens aus A. kawachii

### Herstellung

### 1. ODN-Synthese

Die Synthese sämtlicher Oligonukleotide (ODN) erfolgte nach der Phosphoramidit-Methode an einem Expedite 8909 Synthesizer (ehem. Perseptive Biosystems). Alle verwendeten Chemikalen wurden über die Firma Proligo (Harnburg) bezogen. Die verwendeten Amidite wurden in trockenem Acetonitril (Proligo) aufgenommen (alle Bausteine, einschließlich des Phosphorylierungsreagenzes, in einer Endkonzentration von 0.1 M) und vor Ihrem Einsatz über aktiviertem Molekularsieb (Merck) getrocknet. Um eine möglichst effiziente Synthese von besonders langen Oligonukleotiden zu ermöglichen, wurden alle Kupplungszeiten auf 3 Minuten verlängert. Als Aktivator für die Kupplungsreaktion diente Dicyanoimidazol (Proligo). Das verwendete CPG-Trägermaterial wies dabei eine Porenweite von 1000Å (Länge <130bp, Proligo) bzw. O 2000Å (Länge > 130bp, Glen Research) auf. Um möglichst vollständig 5'-phosphorylierte ODN's zu erhalten, wurde nach der DMTr-on Synthese das 5' Ende mit Hilfe von [3-(4,4'-Dimethoxytrityloxy)-2 ,2' -dicarboxyethyl]propyl-(-2-cyanoethyl)-(N, N'-diisopropyl)-phosphoramidit (CPRII, Glen Research) umgesetzt. Um auch in diesem Fall eine entsprechend hohe Kupplungsausbeute zu erhalten, wurden die Kupplungszeiten für diese Verbindung auf 30 Minuten verlängert. Insgesamt wurden so für die Xylanase 7 ODN's (Xyl1-Xyl7), darunter 6 5'-terminal phosphorylierte (Xyl2-Xyl7), mit einer durchschnittlichen Länge von 70-90b aufgebaut (Tabelle 1). Für die Synthese der Scharnier-DNA-Oligonukleotide (Übergangsstücke ÜXyl1-6) wurden die Syntheseprotokolle nicht weiter modifiziert.

### 2. Aufreinigung

Nach der Synthese erfolgte die Entschützung der Basenschutzgruppen. Hierzu wurde das Trägermaterial (etwa 7mg CPG) in ein verschraubbares Gefäß überführt und 24h bei 37°C mit einer Lösung (500µl) bestehend aus drei Teilen 32%igem Ammoniak (Merck) und einem Teil abs. Ethanol (Fluka) behandelt. Nach erfolgter Abspaltungsreaktion läßt man den Ansatz auf Eis abkühlen und versetzt die Mischung mit 100µl einer 1M Triethylammoniumacetat-Lösung (TEAA). Die gesamte Probe wird anschließend durch Filtration vom Trägermaterial befreit und über RP-HPLC aufgereinigt (Säule: 4.6mm x 300mm gepackt mit POROS R2 (Perseptive Biosystems); Puffer A: 100mM TEAA, 5% Acetonitril; Puffer B: Acetonitril; Fluß: 4ml/min; Gradient: 40 Säulenvolumina von 0% bis 50% Puffer B). Die Hauptfraktionen wurden aufgefangen und unter Vakuum getrocknet. Nach Detritylierung mit 80% Essigsäure (30 Minuten bei 22°C) wurde die Essigsäure unter Vakuum abgezogen und der verbliebene Rückstand zur Abspaltung der Phosphatschutzgruppe 15 Minuten mit 300µl wässriger Ammoniaklösung (2 Teile dest. Wasser/konz. Ammoniak) behandelt. Da das erste Basis-DNA-Oligonukleotid Xyl1 keine terminale Modifikation (5' Phosphat) aufweist, entfiel die basische Behandlung. Anschließend wurden die fertig entschützten Oligonukleotide mit Ethanol gefällt, in dest. Wasser aufgenommen und über denaturiende PAGE (15%) analysiert. Die Sichtbarmachung der Oligonukleotide erfolgte durch Silberfärbung. Verkürzte Sequenzen konnten hierbei für alle Oligonukleotide nicht nachgewiesen werden.

### 3. Gensynthese (Überblick)

Die Gensynthese läßt sich in zwei Teilschritte unterteilen. Zunächst findet ein Ligationsschritt statt, bei dem die Basis-DNA-Oligonukleotide (Xyl1-Xyl7, Tabelle 1) nach Hybridisierung an die kurzen Scharnier-DNA-Oligonukleotide (Übergangsstücke ÜXyl1-ÜXyl6) mit Hilfe einer Ligase (z.B. *Taq*-Ligase, T4-DNA-Ligase oder *E. coli* Ligase) miteinander verknüpft werden. Dieser Teilschritt wird hier allgemein als oligonukleotidspezifische Ligation (OSL) bezeichnet. Nach der OSL wird der gesamte Reaktionsansatz mit Exonuklease VII behandelt. Hierbei werden sämtliche nicht-eingebauten Oligonukleotide, einschließlich der Scharnier-DNA-Oligonukleotide hydrolysiert. Ein kleiner Teil des Hydrolyseansatzes wird anschließend in einer PCR mit zwei terminal zu den ODN's Xyl1 und Xyl7 bindenden Primern (APXyl1 und APXyl7), eingesetzt. Diese Reaktion liefert spezifisch das Xylanase Gen und ermöglicht gleichzeitig durch die mit APXyl1 sowie APXyl7 eingeführten Linkersequenzen eine Klonierung in ein entsprechendes Plasmid.

### 4. Oligonukleotidspezifische Ligation (OSL)

Für die TSL wurden je 2µl der ODN's Xyl1-Xyl7 (10µM) sowie 10µl der Scharnier-DNA-Oligonukleotide ÜXyl1-ÜXyl6 (10µM) in einem Reaktionsgefäß zusammengegeben. Anschließend wurde dieser Ansatz mit 8.2µl 10xLigase Puffer (New England Biolabs) vermischt und mit 2µl (80U) *Taq*-DNA Ligase (New England Biolabs) versetzt. Die weitere Inkubation erfolgte bei 37°C für 12-14h.

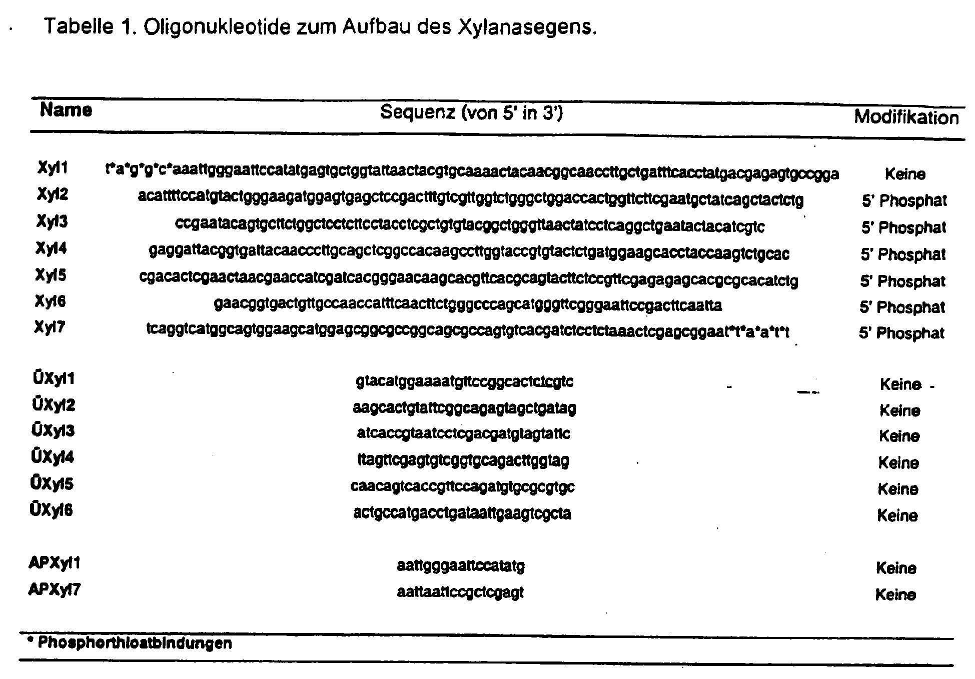

### 5. Exonukleasebehandlung

Der gesamte Ligationsansatz wurde zunächst mit 50µl 3M Natriumacetat (pH 5.2) und 500µl abs. Ethanol auf Eis gefällt. Der Rückstand wird nach der Fällung unter Vakuum getrocknet und in 50µl dest. Wasser gelöst. Zu diesem Ansatz wurden 50µl Exonuklease VII (20U, Pharmacia Biotech) in 100mM Tris-HCl pH8.0, 400mM NaCl zugegeben und das Ganze 45 Minuten bei 37°C inkubiert. Der Nukleaseansatz wurde anschließend 1 x mit Phenol-Chloroform bzw. 2 x mit Chloroform ausgeschüttelt und der wässrige Überstand in ein steriles Cap überführt.

### 6. PCR

Zur gezielten Amplifikation des so aufgebauten, einzelsträngigen Gens wurde 2µl des Nukleaseansatzes mit je 10µl der Außenprimer APXyl1 (10µM) und APXyl7 (10µM), 8µl dNTP-Mix (1,25mM/dNTP), 5µl 10x Polymerase Puffer (New England Biolabs) und 13µl dest. Wasser versetzt, vermischt und für 5 Minuten auf 95°C erhitzt. Danach wurde die Mischung auf Eis abgeschreckt, mit 2µl (4U) Vent Polymerase (New England Biolabs) versetzt und bei 40°C (Anlagerung) in den Thermocycler (Hybaid) gestellt. Die anschließende Amplifikation des Xylanasegens erfolgte unter folgenden Bedingungen:

**Tabelle 2: PCR-Bedingungen**

| **Schritt** | **Temperatur** | **Zeit** |
|---|---|---|
| Anlagerung | 40°C | 30sec |
| Verlängerung | 72°C | 1min |
| Denaturierung | 95°C | 30sec |

| | | |
|---|---|---|
| **Zyklenzahl 35** | | |

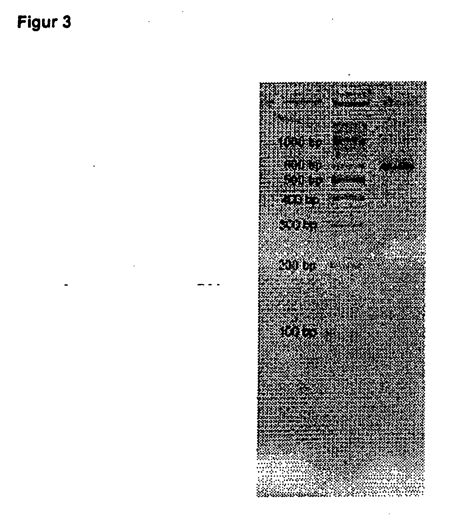

Nach Ablauf der PCR wurde der gesamte Reaktionsansatz mit 5x Probenpuffer versetzt und über Gelelektrophorese (3% Agarose) aufgereinigt (Figur 3). Die Isolierung des Xylanase Gens erfolgte nach Elektroelution der aus dem Gel ausgeschnittenen Agarosebande (Sambrook et al., 1989, Molecular Cloning-A Laboratory Manual).

### 7. Klonierung

Das nach Elution und Extraktion (Sambrook et al., 1989, Molecular Cloning-A Laboratory Manual) in TE Puffer (10mM Tris-HCl, 0.5mM EDTA pH8.0) aufgenommene Xylanase Gen wurde vollständig mit den Restriktionsenzymen Ndel (New England Biolabs) und Xhol (New England Biolabs) über Nacht bei 37°C verdaut und über Gelelektrophorese erneut isoliert. Nach Elektroelution und Aufarbeitung des Fragmentes wurde dieses über Nacht bei 16°C in den entsprechend geöffneten pET23a Vektor (Novagen) einligiert. Zur Ligation wurden 200U T4 DNA Ligase eingesetzt. 5µl des Ligationsansatzes wurden anschließend in kompetente Zellen (DH5α) transformiert (Sambrook et al., 1989, Molecular Cloning-A Laboratory Manual).

### 8. Sequenzierung

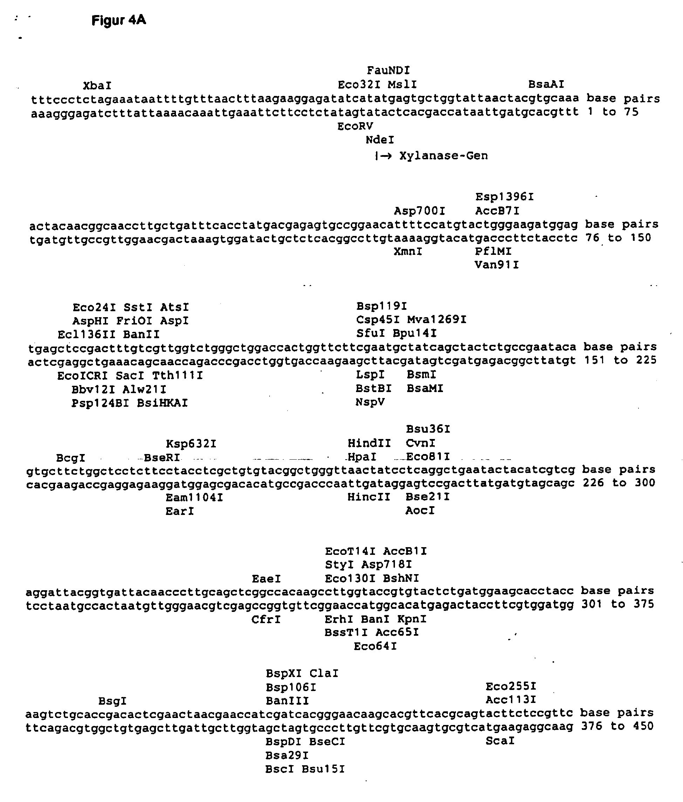

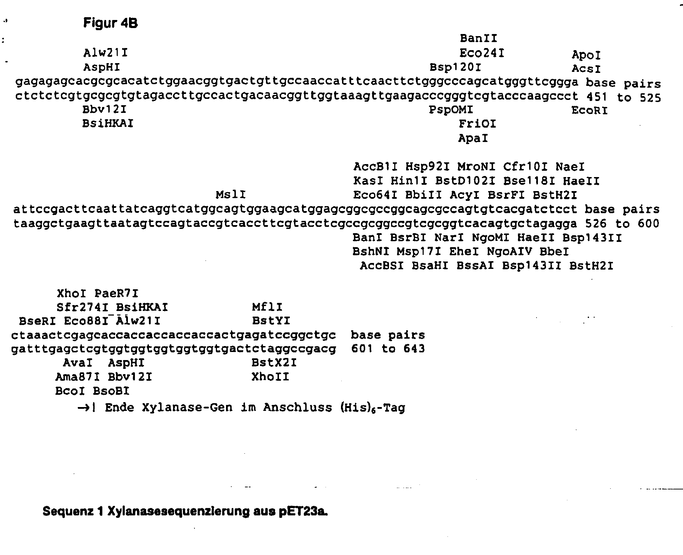

Die durchgeführte Sequenzierung nach Saenger bei einem willkürlich isolierten Klon, erbrachte die vollständige Übereinstimmung mit der entworfenen Sequenz (Figur 4A und 4B).

### Beispiel 2: Synthese des Gens für humanes Chymotrypsinogen A

### 1. ODN Synthese

Die Synthese sämtlicher Oligonukleotide (ODN) erfolgte nach der Phosphoramidit-Methode an einem Expedite 8909. Alle verwendeten Chemikalien und Syntheseprotokolle entsprechen den Angaben aus Abschnitt 1 von Beispiel 1. Zum Aufbau der Chymotrypsinogen-DNA wurden insgesamt 11 ODN's (Ch1-Ch11), darunter 10 5'-terminal phosphorylierte (Ch2-Ch11), mit einer durchschnittlichen Länge von 90b aufgebaut (Tabelle 3). Auch in diesem Beispiel wurden die Syntheseprotokolle zur Synthese der kurzen Übergangsstücke (ÜCh1-10) nicht weiter modifiziert.

### 2. Aufreinigung

Nach der Synthese erfolgte die Entschützung der Basenschutzgruppen. Hierzu wurde das Trägermaterial (etwa 7mg CPG) in ein verschraubbares Gefäß überführt und 24h bei 37°C mit einer Lösung (500µl) bestehend aus drei Teilen 32%igem Ammoniak (Merck) und einem Teil abs. Ethanol (Fluka) behandelt. Nach erfolgter Abspaltungsreaktion läßt man den Ansatz auf Eis abkühlen und versetzt die Mischung mit 100µl einer 1M Triethylammoniumacetat-lösung (TEAA). Die gesamte Probe wird anschließend durch Filtration vom Trägermaterial befreit und über RP-HPLC aufgereinigt (Säule: 4.6mm x 300mm gepackt mit POROS R2 (Perseptive Biosystems); Puffer A: 100mM TEAA, 5% Acetonitril; Puffer B: Acetonitril; Fluß: 4ml/min; Gradient: 40 Säulenvolumina von 0% bis 50% Puffer B). Die Hauptfraktionen wurden aufgefangen und unter Vakuum getrocknet. Nach Detritylierung mit 80%iger Essigsäure (30 Minuten bei 22°C) wurde erneut bis zur Trockene einrotiert und zur Abspaltung der Phosphatschutzgruppe der Rückstand 15 Minuten mit 300µl wässriger Ammoniaklösung (2 Teile dest. Wasser/konz. Ammoniak) behandelt. Anschließend wurde das fertig entschützte Oligonukleotid mit Ethanol gefällt, in dest. Wasser aufgenommen und über denaturierende PAGE analysiert.

### 3. Gensynthese

Die hier durchgeführte Totalsynthese des Gens für Chymostrypsinogen A läßt sich genau wie beim Xylanase-Gen in zwei Teilschritte unterteilen. Zunächst werden durch OSL die langen Basis-DNA-Oligonukleotide (Ch1-11, Tabelle 3) miteinander verknüpft . Nach der OSL wird der gesamte Reaktionsansatz mit Exonuklease VII behandelt. Hierbei werden sämtliche nicht-eingebauten Oligonukleotide, einschließlich der Scharnier-DNA-Oligonukleotide hydrolysiert. Ein kleiner Teil des Hydrolyseansatzes wird anschließend in einer PCR mit zwei terminal zu den ODN's Ch1 und Ch11 bindenden Primern (APCh1 und APCh11) eingesetzt. Diese Reaktion liefert auch hier spezifisch das Chymotrypsinogen A-Gen.

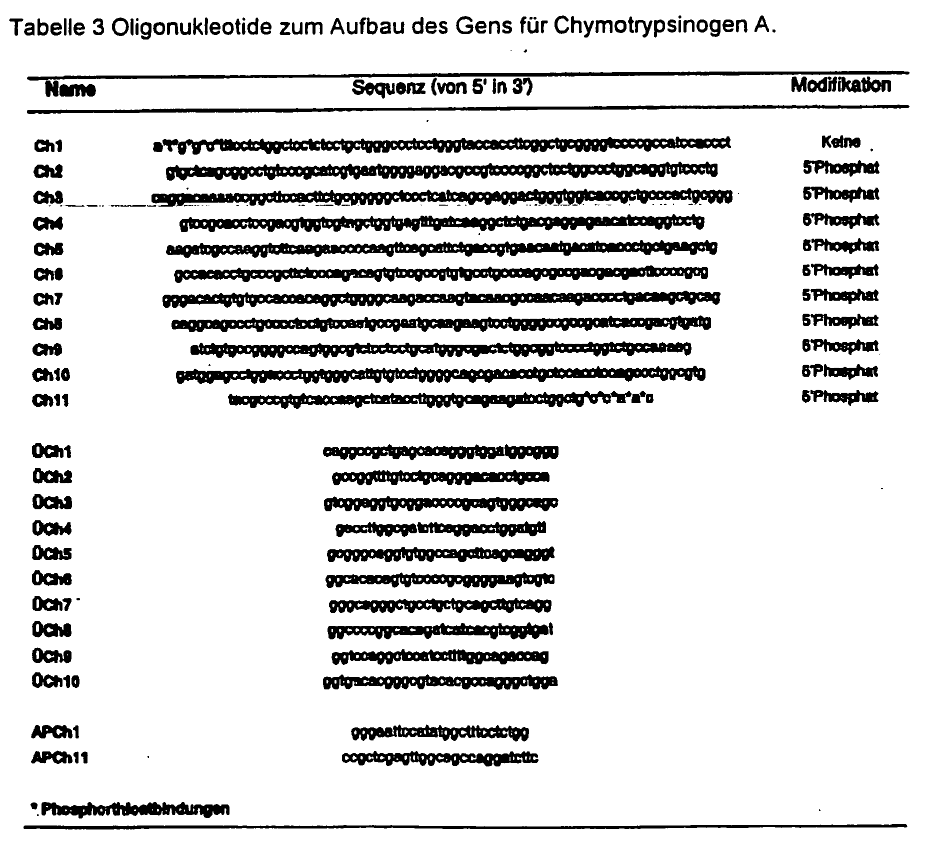

### 4. Oligonukleotidspezifische Ligation (OSL)

Für die TSL wurden je 0.8µl der ODN's Ch1-Ch11 (10µM) sowie 4µl der Scharnier-DNA-Oligonukleotide ÜCh1-ÜCh10 (10µM) in einem Reaktionsgefäß zusammengegeben. Alternativ kann auch ein Mischungsverhältnis von 1:1 bis 1:10 verwendet werden. Anschließend wurde dieser Ansatz mit 8.2µl 10xLigase-Puffer (New England Biolabs) vermischt und mit 2µl (8U) Taq-DNA Ligase (New England Biolabs) versetzt und auf 80µl mit dest. Wasser aufgefüllt. Die weitere Inkubation erfolgte bei 37°C für 12-14h.

### 5. Exonukleasebehandlung

Entsprechend Beispiel 1 wurde der gesamte Ligationsansatz zunächst mit 50µl 3M Natriumacetat (pH 5.2) und 500µl abs. Ethanol auf Eis gefällt. Der Rückstand wird nach der Fällung unter Vakuum getrocknet und in 50µl dest. Wasser gelöst. Zu diesem Ansatz wurden 50µl Exonuklease VII (20U) in 100mM Tris-HCl pH8.0, 400mM NaCl zugegeben und das Ganze 45Minuten bei 37°C inkubiert. Der Nukleaseansatz wurde anschließend mit Phenol-Chloroform bzw. Chloroform ausgeschüttelt und der wässrige Überstand in ein steriles Cap überführt.

### 6. PCR

Zur Amplifikation des über OSL zusammengebauten Gens wurde 2µl des Nukleaseansatzes mit je 10µl der Außenprimer APCh1 (10µM) und APCh11 (10µM), 8µl dNTP-Mix (1.25mM/dNTP), 5µl 10xPolymerase-Puffer (New England Biolabs) und 13µl dest. Wasser versetzt, vermischt und für 5 Minuten auf 95°C erhitzt. Danach wurde die Mischung auf Eis abgeschreckt, mit 2µl (4U) Vent Polymerase (New England Biolabs) versetzt und bei 54°C (Anlagerung) in den Cycler (Hybaid) gestellt. Die Amplifikation des Chymotrypsinogen-Gens erfolgte unter folgenden Bedingungen:

**Tabelle 4: PCR-Bedingungen**

| **Schritt** | **Temperatur** | **Zeit** |
|---|---|---|
| Anlagerung | 54°C | 30sec |
| Verlängerung | 72°C | 1.5min |
| Denaturierung | 94°C | 30sec |

| | | |
|---|---|---|
| **Zyklenzahl 35** | | |

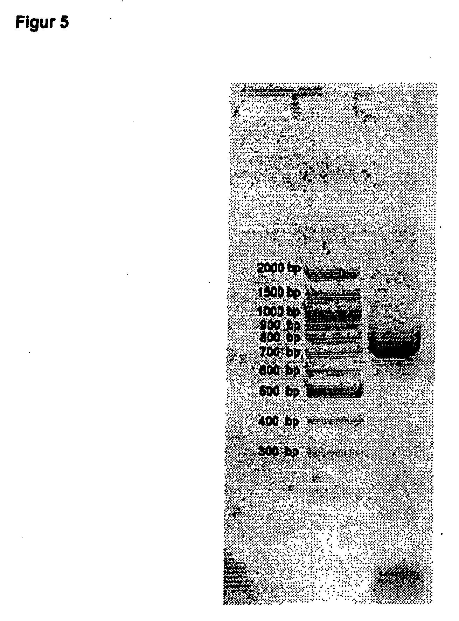

Nach Ablauf der PCR wurde der gesamte Reaktionsansatz mit 5xProbenpuffer versetzt und über Gelelektrophorese (1.5% Agarose) aufgereinigt (Figur 5). Die Isolierung der Chymotrypsinogen-DNA erfolgte nach Elektroelution der aus dem Gel ausgeschnittenen Agarosebande (Sambrook et al., 1989, Molecular Cloning-A Laboratory Manual).

### 7. Klonierung

Die nach Elution und Extraktion (Sambrook et al., 1989, Molecular Cloning-A Laboratory Manual) in TE Puffer (10mM Tris-HCl, 0,5mM EDTA pH8.0) aufgenommene Chymotrypsinogen-DNA wurde vollständig mit den Restriktionsenzymen Ndel (New England Biolabs) und Xhol (New England Biolabs) über Nacht bei 37°C verdaut und über Gelelektrophorese erneut isoliert. Nach Elektroelution und Aufarbeitung des Fragmentes konnte dieses über Nacht bei 16°C in den entsprechend geöffneten pET23a Vektor (Novagen) einligiert werden. Zur Ligation wurden 200U T4 DNA Ligase (New England Biolabs) eingesetzt. 10µl des Ligationsansatzes wurden anschließend in kompetente Zellen (DH5α) transformiert (Sambrook et al., 1989, Molecular Cloning-A Laboratory Manual).

### 8. Sequenzierung

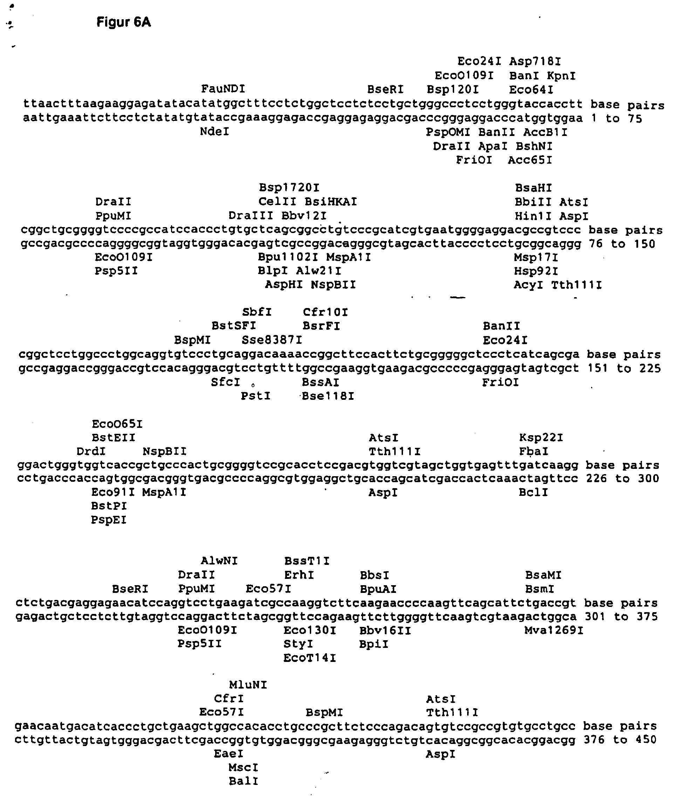

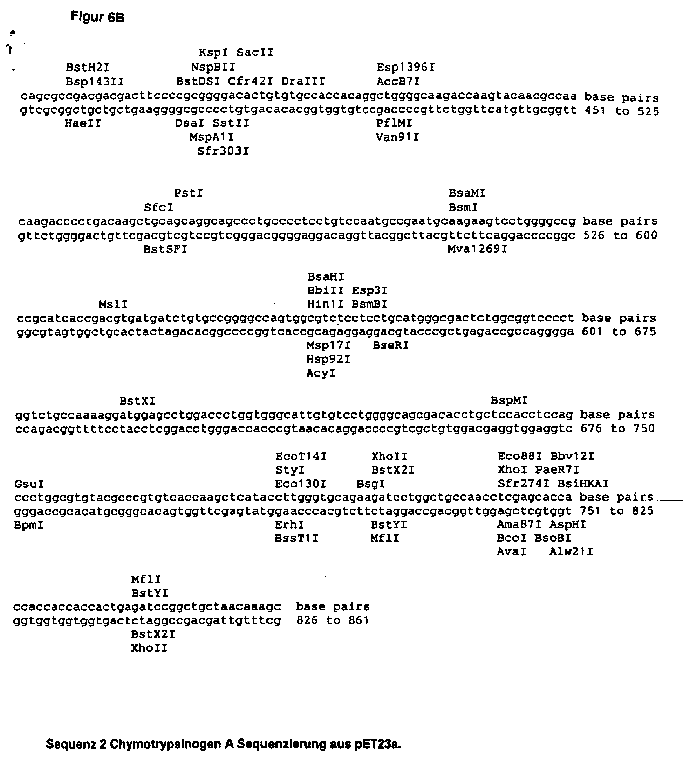

Die durchgeführte Sequenzierung nach Saenger bei einem willkürlich isolierten Klon erbrachte die vollständige Übereinstimmung mit der entworfenen Sequenz (Figur 6A und 6B).

### Zitierte Veröffentlichungen

Au, L.C. et al. (1998) Biochem. Biophys. Res. Commun., 248,200-203
Ausubel, F.M. et al. (1994) Current protocols in molecular biology, Vol.I, Wiley
Casimiro, D.R. et al. (1997) Structure, 5,1407-1412
Chalmers, F.M. & Curnow, K.M. BioTechniques, 30,249-252
Chang, H.H. (1993) Biochem. Biophys. Res. Commun., 190,242-249
Chen, H.B. (1990) Nucleic Acids Res., 18, 871-878
Ciccarelli, R.B. et al. (1991) Nucl. Acids Res., 19,6007-6013
Climie, S. et al. (1990) Proc. Natl. Acad. Sci. USA, 87,633-637
Dillon, P.J. & Rosen, C.A. (1990) BioTechniques, 9,298-300
Ferretti, L. (1986) Proc. Natl. Acad. Sci.USA, 83,599-603
Hostomsky, Z. et al. (1987) Nucl. Acids Res., 15,4849-4856
Jayaraman, K. et al. (1991) Proc. Natl. Acad. Sci.USA, 88,4084-4088
Jayaraman, K. & Puccini, C.J. (1992) BioTechniques, 12, 392-398
Oprian, D.D. et al. (1991) Biochemistry, 30, 11367 -11372
Spoat, B.S. et al. (1985) Nucl. Acids Res., 13,2959-2977
Traub, P.C. et al. (2001) Appl. Microbiol. Biotechnol. (2001), 55, 198-204
Uhlmann, E. & Hein, F. (1987) NucleicAcids Symp Ser, 18,237-240
Wosnick, M.A. (1989) Gene, 76,153-160
Ye, Q.Z. (1992) Biochem. Biophys. Res. Commun., 186,143-149

## Patentansprüche

1. Verfahren zur Herstellung von DNA, das umfasst, daß man
a) n einzelsträngige Basis-DNA-Oligonukleotide bereitstellt, die unmittelbar aufeinanderfolgende Teile der Nukleotidsequenz der herzustellenden DNA sind, wobei
i) das zweite bis n-te Basis-DNA-Oligonukleotid am 5'-Ende phosphoryliert ist und ii) n 3 bis 100 ist;
b) wenigstens (n-1) einzelsträngige Scharnier-DNA-Oligonukleotide bereitstellt, wobei für die Scharnier-DNA-Oligonukleotide gilt, daß der 3'-terminale Bereich eines Scharnier-DNA-Oligonukleotids wenigstens teilweise komplementär zum 3'-terminalen Bereich eines Basis-DNA-Oligonukleotids ist, und der 5'-terminale Bereich desselben Scharnier-DNA-Oligonukleotids wenigstens teilweise komplementär zum 5'-terminalen Bereich des unmittelbar darauffolgenden Basis-DNA-Oligonukleotids ist, so daß im Falle der Hybridisierung eines Scharnier-DNA-Oligonukleotids mit 2 unmittelbar aufeinanderfolgenden Basis-DNA-Oligonukleotiden ein im Bereich des Scharnier-DNA-Oligonukleotids doppelsträngiges DNA-Hybrid entsteht;
c) die Basis-DNA-Oligonukleotide mit den Scharnier-DNA-Oligonukleotiden in Kontakt bringt;
d) das aus Schritt c) resultierende DNA-Hybrid einer Ligationsreaktion unterwirft;
e) das Reaktionsprodukt aus Schritt d) einer Exonukleasereaktion unterwirft, wobei der durch ligierte Basis-DNA-Oligonukleotide gebildete DNA-Strang des Reaktionsprodukts aus Schritt d) wenigstens zwei Cap-Strukturen umfasst, wobei das Reaktionsprodukt der Exonukleasebehandlung einzelsträngige DNA mit Cap-Strukturen an ihren Enden ist, und wobei eine Cap-Struktur eine Struktur ist, die einem Ende einer linearen Nukleinsäure Resistenz gegen eine Exonuklease verleiht.

2. Verfahren nach Anspruch 1, **dadurch gekennzeichnet, daß** das Reaktionsprodukt aus Schritt e) weiterhin einer PCR unterworfen wird.

3. Verfahren nach Anspruch 2, **dadurch gekennzeichnet, daß** in der PCR ein erster Primer verwendet wird, dessen Zielsequenz im Bereich des ersten Basis-DNA-Oligonukleotids liegt, und ein zweiter Primer verwendet wird, dessen Zielsequenz im Bereich des n-ten Basis-DNA-Oligonukleotids liegt.

4. Verfahren nach Anspruch 2 oder 3, **dadurch gekennzeichnet, daß** in der PCR Primer eingesetzt werden, die eine oder mehrere Erkennungssequenzen für eine oder mehrere Restriktionsendonukleasen enthalten.

5. Verfahren nach einem der Ansprüche 2 bis 4, **dadurch gekennzeichnet, daß** das doppelsträngige Reaktionsprodukt der PCR weiterhin einem Restriktionsverdau unterworfen wird.

6. Verfahren nach einem der vorhergehenden Ansprüche, **dadurch gekennzeichnet, daß** die Ligationsreaktion mittels einer Ligase, die gegebenenfalls thermostabil ist, durchgeführt wird.

7. Verfahren nach einem der vorhergehenden Ansprüche, **dadurch gekennzeichnet, daß** die Ligationsreaktion mittels einer Ligase durchgeführt wird, die ausgewählt ist aus der Gruppe bestehend aus T4-DNA-Ligase, *Taq* DNA-Ligase und *Pfu* DNA-Ligase.

8. Verfahren nach einem der vorhergehenden Ansprüche, **dadurch gekennzeichnet, daß** die Exonukleasereaktion mittels eines Enzyms durchgeführt wird, das ausgewählt ist aus der Gruppe bestehend aus Exonuklease VII, allgemein Exonukleasen, jedoch auch Exonuklease I, Exonuklease III und Exonuklease V als auch DNase I sowie Mischungen der soeben beschriebenen Hydrolasen.

9. Verfahren nach einem der vorhergehenden Ansprüche, **dadurch gekennzeichnet, daß** die Cap-Struktur, die einem Ende einer linearen Nukleinsäure Resistenz gegen eine Exonuklease verleiht, ausgewählt ist aus der Gruppe bestehend aus Thioatbindungen zwischen einzelnen Nukleotiden, 2'OMethyl-RNA, modifizierten Basen, die Schutz gegen Exonuklease-Abbau verleihen, DNA-Sequenzen mit Schleifenstruktur(en) und RNA-Sequenzen mit Schleifenstruktur(en).

10. Verfahren nach einem der vorhergehenden Ansprüche, **dadurch gekennzeichnet, daß** das erste Basis-DNA-Oligonukleotid eine Cap-Struktur umfaßt und das n-te Basis-DNA-Oligonukleotid eine Cap-Struktur umfaßt.

11. Verfahren nach einem der vorhergehenden Ansprüche, **dadurch gekennzeichnet, daß** die Basis-DNA-Oligonukleotide und/oder die Scharnier-DNA-Oligonukleotide durch die Phosphoramidit-Methode hergestellt werden.

12. Verfahren nach einem der vorhergehenden Ansprüche, **dadurch gekennzeichnet, daß** ein oder mehrere Basis-DNA-Oligonukleotide und/oder Scharnier-DNA-Oligonukleotide randomisiert Nukleotide enthalten.

13. Verfahren nach einem der vorhergehenden Ansprüche, **dadurch gekennzeichnet, daß** die hergestellte DNA weiterhin in einen Vektor oder ein Plasmid kloniert wird.

14. Verfahren nach einem der vorhergehenden Ansprüche, **dadurch gekennzeichnet, daß** die hergestellte DNA oder der hergestellte Vektor oder das hergestellte Plasmid in eine Zelle eingeführt wird, mit der Maßgabe, dass die Einführung der DNA oder des Vektors in die Zelle nicht *in vivo* durchgeführt wird.

15. DNA-Hybrid umfassend einen Einzelstrang, einen oder mehrere damit hybridisierende Scharnier-DNA-Oligonukleotide, eine Cap-Struktur im 5'-terminalen Bereich des Einzelstrangs und eine Cap-Struktur im 3'-terminalen Bereich des Einzelstrangs.

16. Kit zur Herstellung von DNA enthaltend ein erstes Basis-DNA-Oligonukleotid, das eine Cap-Struktur umfaßt, ein weiteres Basis-DNA-Oligonukleotid, das eine Cap-Struktur umfaßt, ein Enzym mit Ligaseaktivität und ein Enzym mit Exonukleaseaktivität.

17. Kit nach Anspruch 16, **dadurch gekennzeichnet, daß** es weiterhin Mittel zur Durchführung einer PCR enthält.

18. Kit nach Anspruch 17, **dadurch gekennzeichnet, daß** eine thermostabile DNA-Polymerase und Primer enthält, die eine oder mehrere Erkennungssequenzen für eine oder mehrere Restriktionsendonukleasen enthalten.

## Claims

1. A method for manufacturing DNA comprising the steps of
a) providing n single stranded base-DNA-oligonucleotides which form immediately consecutive parts of the nucleotide sequence of the DNA being manufactured, in which
(i) the second to the n-th base-DNA-oligonucleotide is phosphorylated at the 5'end and
(ii) n is from 3 to 100;
b) providing at least (n-1) single stranded joint-DNA-oligonucleotides, applicable to which joint-DNA-oligonucleotide the 3'terminal region of a joint-DNA-oligonucleotide is at least partially complementary to the 3'terminal region of a base-DNA-oligonucleotide, and the 5'terminal region of the same joint-DNA-oligonucleotide is at least partially complementary to the 5'terminal region of the immediately following base-DNA-oligonucleotide, so that when a joint-DNA-oligonucleotide is hybridized with 2 immediately consecutive base-DNA-oligonucleotides a double-stranded DNA hybrid is formed in the region of the joint-DNA-oligonucleotide;
c) contacting the base-DNA-oligonucleotides with the joint-DNA-oligonucleotides;
d) subjecting the product DNA-hybrid from step c) to a ligation reaction;
e) subjecting the reaction product from step d) to an exonuclease reaction, wherein the DNA strand of the reaction product of step d) formed by ligated base-DNA-oligonucleotides includes at least two cap-structures, and wherein the reaction product of the exonuclease treatment is a single stranded DNA with cap-structures at each end, and wherein the cap-structure is a structure which confers exonuclease resistance on an end of a linear nucleic acid.

2. The method according to claim 1, **characterized in that** the reaction product from step e) is further subjected to a PCR.

3. The method according to claim 2, **characterized in that** in said PCR a first primer is used that has a target sequence located in the region of the first base-DNA-oligonucleotide, and a second primer is used that has a target sequence located in the region of the n-th base-DNA-oligonucleotide.

4. The method according to claim 2 or 3, **characterized in that** in said PCR primers are used which contain one or more recognition sequences for one or more restriction endonucleases.

5. The method according to any one of claims 2 to 4, **characterized in that** the double stranded reaction product of the PCR is further subjected to restriction digestion.

6. The method according to any one of the preceding claims, **characterized in that** the ligation reaction is carried out using a ligase which may be thermostable.

7. The method according to any one of the preceding claims, **characterized in that** the ligation reaction is carried out using a ligase selected from the group consisting of T4-DNA-ligase, *Taq* DNA-ligase and *Pfu* DNA-ligase.

8. The method according to any one of the preceding claims, **characterized in that** the exonuclease reaction is carried out using an enzyme selected from the group consisting of exonuclease VII, exonucleases in general, but also exonuclease I, exonuclease III and exonuclease V, as well as DNase 1 and mixtures of the aforementioned hydrolases.

9. The method according to any one of the preceding claims, **characterized in that** the cap-structure, which confers exonuclease resistance on an end of a linear nucleic acid, is selected from the group consisting of thioate bonds between individual nucleotides, 2'Omethyl-RNA, modified bases which confer protection against exonuclease degradation, DNA-sequences with loop structure(s) and RNA-sequences with loop structure(s).

10. The method according to any one of the preceding claims, **characterized in that** said first base-DNA-oligonucleotide includes a cap-structure and said n-th base-DNA-oligonucleotide includes a cap-structure.

11. The method according to any one of the preceding claims, **characterized in that** said base-DNA-oligonucleotides and/or joint-DNA-oligonucleotides are produced by way of the phosphoramidite method.

12. The method according to any one of the preceding claims, **characterized in that** one or more base-DNA-oligonucleotides and/or joint-DNA-oligonucleotides contain randomized nucleotides.

13. The method according to any one of the preceding claims, **characterized in that** the manufactured DNA is further cloned in a vector or a plasmid.

14. The method according to any one of the preceding claims, **characterized in that** the manufactured DNA or the manufactured vector or the manufactured plasmid is introduced into a cell, with the proviso that the DNA or the vector is not introduced into the cell *in vivo.*

15. A DNA hybrid comprising a single strand, one or more joint-DNA-oligonucleotides hybridized therewith, a cap-structure in the 5'terminal region of the single strand and a cap-structure in the 3'terminal region of the single strand.

16. A kit for manufacturing DNA, which contains a first base-DNA-oligonucleotide that includes a cap-structure, a second base-DNA-oligonucleotide that includes a cap-structure, an enzyme exhibiting ligase activity and an enzyme exhibiting exonuclease activity.

17. A kit according to claim 16, **characterized in that** said kit also contains means for performing a PCR.

18. A kit according to claim 17, **characterized in that** said kit contains a thermostable DNA-polymerase and primers which contain one or more recognition sequences for one or more restriction endonucleases.

## Revendications

1. Procédé de production d'ADN, comportant :
a) la préparation de n oligonucléotides d'ADN de base simple brin qui sont des parties successives de la séquence de nucléotides de l'ADN à produire,
i) les deuxième à n^{ième} oligonucléotides de l'ADN de base étant phosphorylés à l'extrémité 5' et
ii) n allant de 3 à 100 ;
b) la préparation d'au moins (n-1) oligonucléotides d'ADN charnière simple brin, les oligonucléotides de l'ADN charnière étant tels que la région 3'-terminale d'un oligonucléotide de l'ADN charnière est au moins partiellement complémentaire de la région 3'-terminale d'un oligonucléotide de l'ADN de base, et la région 5'-terminale de ce même oligonucléotide de l'ADN charnière est au moins partiellement complémentaire de la région 5'-terminale de l'oligonucléotide immédiatement suivant de l'ADN de base de sorte que, en cas d'hybridation d'un oligonucléotide de l'ADN charnière avec 2 oligonucléotides successifs de l'ADN de base, on obtient un hybride d'ADN double brin dans la région du oligonucléotide de l'ADN charnière ;
c) la mise en contact des oligonucléotides de l'ADN de base avec les oligonucléotides de l'ADN charnière ;
d) la soumission de l'hybride d'ADN résultant de l'étape c) à une réaction de ligature ;
e) la soumission du produit de la réaction de l'étape d) à une réaction avec une exonucléase, le brin d'ADN, formé par des oligonucléotides ligaturés de l'ADN de base, du produit de la réaction de l'étape d) comportant au moins deux structures coiffe, le produit de la réaction avec l'exonucléase étant des ADN simple brin ayant une structure coiffe à leur extrémité et une structure coiffe étant une structure qui confère à une extrémité d'un acide nucléique linéaire une certaine résistance à une exonucléase.

2. Procédé selon la revendication 1, **caractérisé en ce que** le produit de la réaction de l'étape e) est soumis en outre à une PCR.

3. Procédé selon la revendication 2, **caractérisé en ce que** l'on utilise dans la PCR une première amorce dont la séquence cible se trouve dans la région du premier oligonucléotide de l'ADN de base, et **en ce que** l'on utilise une deuxième amorce dont la séquence cible se trouve dans la région du n^{ième} oligonucléotide de l'ADN de base.

4. Procédé selon la revendication 2 ou 3, **caractérisé en ce que** l'on utilise dans la PCR des amorces qui contiennent une ou plusieurs séquences de reconnaissance d'une ou plusieurs endonucléases de restriction.

5. Procédé selon l'une des revendications 2 à 4, **caractérisé en ce que** le produit double brin de la réaction PCR est soumis en outre à une digestion de restriction.

6. Procédé selon l'une des revendications précédentes, **caractérisé en ce que** la réaction de ligature est réalisée au moyen d'une ligase qui est, le cas échéant, thermostable.

7. Procédé selon l'une des revendications précédentes, **caractérisé en ce que** la réaction de ligature est réalisée au moyen d'une ligase qui est sélectionnée dans le groupe constitué par la T4-ADN-ligase, la *Taq* ADN-ligase et la *Pfu* ADN-ligase.

8. Procédé selon l'une des revendications précédentes, **caractérisé en ce que** la réaction avec une exonucléase est réalisée au moyen d'une enzyme qui est sélectionnée dans le groupe constitué par l'exonucléase VII, les exonucléases en général, mais également l'exonucléase I, l'exonucléase III et l'exonucléase V, ainsi que l'ADNase I et des mélanges des hydrolases susmentionnées.

9. Procédé selon l'une des revendications précédentes, **caractérisé en ce que** la structure coiffe, qui confère à une extrémité d'un acide nucléique linéaire une certaine résistance à une exonucléase, est sélectionnée dans le groupe constitué par les liaisons thioate entre des nucléotides individuels, le 2'-O-méthyl-ARN, des bases modifiées qui confèrent une protection contre la désagrégation par une exonucléase, des séquences d'ADN comportant une ou des structures en boucle et des séquences d'ARN comportant une ou des structures en boucle.

10. Procédé selon l'une des revendications précédentes, **caractérisé en ce que** le premier oligonucléotide de l'ADN de base a une structure coiffe et le n^{ième} oligonucléotide de l'ADN de base a une structure coiffe.

11. Procédé selon l'une des revendications précédentes, **caractérisé en ce que** les oligonucléotides de l'ADN de base et les oligonucléotides de l'ADN charnière sont fabriqués par le procédé au phosphoramidite.

12. Procédé selon l'une des revendications précédentes, **caractérisé en ce qu'**un ou plusieurs oligonucléotides de l'ADN de base et/ou oligonucléotides de l'ADN charnière contiennent des nucléotides randomisés.

13. Procédé selon l'une des revendications précédentes, **caractérisé en ce que** l'ADN produit est en outre cloné dans un vecteur ou un plasmide.

14. Procédé selon l'une des revendications précédentes, **caractérisé en ce que** l'ADN produit ou le vecteur produit ou le plasmide produit est introduit dans une cellule, l'introduction de l'ADN ou du vecteur dans la cellule n'étant pas effectuée *in vivo.*

15. Hybride d'ADN comportant un simple brin, un ou plusieurs oligonucléotides d'ADN charnière s'hybridant avec celui-ci, une structure coiffe dans la région 5'-terminale du simple brin et une structure coiffe dans la région 3'-terminale du simple brin.

16. Kit de production d'ADN contenant un premier oligonucléotide d'ADN de base qui a une structure coiffe, un autre oligonucléotide d'ADN de base qui a une structure coiffe, une enzyme ayant l'activité d'une ligase et une enzyme ayant l'activité d'une exonucléase.

17. Kit selon la revendication 16, **caractérisé en ce qu'**il contient en outre des moyens permettant d'effectuer une PCR.

18. Kit selon la revendication 17, **caractérisé en ce qu'**il contient une ADN-polymérase thermostable et des amorces qui contiennent une ou plusieurs séquences de reconnaissance d'une ou plusieurs endonucléases de restriction.


### SEQUENZPROTOKOLL

<110> BioSpring Gesellschaft für Biotechnologie mbH
<120> Verfahren zur Herstellung von DNA
<130>
<140>
   <141>
<150>
   <151>
<160> 42
<170> PatentIn Ver. 2.1
<210> 1
   <211> 96
   <212> DNA
   <213> künstliche Sequenz
<220>
   <223> Beschreibung der künstlichen Sequenz: DNA
<400> 1
   taggcaaatt gggaattcca tatgagtgct ggtattaact acgtgcaaaa ctacaacggc 60
   aaccttgctg atttcaccta tgacgagagt gccgga 96
<210> 2
   <211> 94
   <212> DNA
   <213> künstliche Sequenz
<220>
   <223> Beschreibung der künstlichen Sequenz: DNA
<400> 2
   acattttcca tgtactggga agatggagtg agctccgact ttgtcgttgg tctgggctgg 60
   accactggtt cttcgaatgc tatcagctac tctg 94
<210> 3
   <211> 83
   <212> DNA
   <213> künstliche Sequenz
<220>
   <223> Beschreibung der künstlichen Sequenz: DNA
<400> 3
   ccgaatacag tgcttctggc tcctcttcct acctcgctgt gtacggctgg gttaactatc 60
   ctcaggctga atactacatc gtc 83
<210> 4
   <211> 86
   <212> DNA
   <213> künstliche Sequenz
<220>
   <223> Beschreibung der künstlichen Sequenz: DNA
<400> 4
   gaggattacg gtgattacaa cccttgcagc tcggccacaa gccttggtac cgtgtactct 60
   gatggaagca cctaccaagt ctgcac 86
<210> 5
   <211> 86
   <212> DNA
   <213> künstliche Sequenz
<220>
   <223> Beschreibung der künstlichen Sequenz: DNA
<400> 5
   cgacactcga actaacgaac catcgatcac gggaacaagc acgttcacgc agtacttctc 60
   cgttcgagag agcacgcgca catctg 86
<210> 6
   <211> 70
   <212> DNA
   <213> künstliche Sequenz
<220>
   <223> Beschreibung der künstlichen Sequenz: DNA
<400> 6
   gaacggtgac tgttgccaac catttcaact tctgggccca gcatgggttc gggaattccg 60
   acttcaatta 70
<210> 7
   <211> 81
   <212> DNA
   <213> künstliche Sequenz
<220>
   <223> Beschreibung der künstlichen Sequenz: DNA
<400> 7
   tcaggtcatg gcagtggaag catggagcgg cgccggcagc gccagtgtca cgatctcctc 60
   taaactcgag cggaattaat t 81
<210> 8
   <211 > 30
   <212> DNA
   <213> künstliche Sequenz
<220>
   <223> Beschreibung der künstlichen Sequenz: DNA
<400> 8
   gtacatggaa aatgttccgg cactctcgtc 30
<210> 9
   <211> 30
   <212> DNA
   <213> künstliche Sequenz
<220>
   <223> Beschreibung der künstlichen Sequenz: DNA
<400> 9
   aagcactgta ttcggcagag tagctgatag 30
<210> 10
   <211> 30
   <212> DNA
   <213> künstliche Sequenz
<220>
   <223> Beschreibung der künstlichen Sequenz: DNA
<400> 10
   atcaccgtaa tcctcgacga tgtagtattc 30
<210> 11
   <211 > 30
   <212> DNA
   <213> künstliche Sequenz
<220>
   <223> Beschreibung der künstlichen Sequenz: DNA
<400> 11
   ttagttcgag tgtcggtgca gacttggtag 30
<210> 12
   <211 > 30
   <212> DNA
   <213> künstliche Sequenz
<220>
   <223> Beschreibung der künstlichen Sequenz: DNA
<400> 12
   caacagtcac cgttccagat gtgcgcgtgc 30
<210> 13
   <211> 30
   <212> DNA
   <213> künstliche Sequenz
<220>
   <223> Beschreibung der künstlichen Sequenz: DNA
<400> 13
   actgccatga cctgataatt gaagtcgcta 30
<210> 14
   <211> 18
   <212> DNA
   <213> künstliche Sequenz
<220>
   <223> Beschreibung der künstlichen Sequenz: DNA
<400> 14
   aattgggaat tccatatg 18
<210> 15
   <211> 18
   <212> DNA
   <213> künstliche Sequenz
<220>
   <223> Beschreibung der künstlichen Sequenz: DNA
<400> 15
   aattaattcc gctcgagt 18
<210> 16
   <211> 78
   <212> DNA
   <213> künstliche Sequenz
<220>
   <223> Beschreibung der künstlichen Sequenz: DNA
<400> 16
   atggctttcc tctggctcct ctcctgctgg gccctcctgg gtaccacctt cggctgcggg 60
   gtccccgcca tccaccct 78
<210> 17
   <211> 75
   <212> DNA
   <213> künstliche Sequenz
<220>
   <223> Beschreibung der künstlichen Sequenz: DNA
<400> 17
   gtgctcagcg gcctgtcccg catcgtgaat ggggaggacg ccgtccccgg ctcctggccc 60
   tggcaggtgt ccctg 75
<210> 18
   <211> 78
   <212> DNA
   <213> künstliche Sequenz
<220>
   <223> Beschreibung der künstlichen Sequenz: DNA
<400> 18
   caggacaaaa ccggcttcca cttctgcggg ggctccctca tcagcgagga ctgggtggtc 60
   accgctgccc actgcggg 78
<210> 19
   <211 > 72
   <212> DNA
   <213> künstliche Sequenz
<220>
   <223> Beschreibung der künstlichen Sequenz: DNA
<400> 19
   gtccgcacct ccgacgtggt cgtagctggt gagtttgatc aaggctctga cgaggagaac 60
   atccaggtcc tg 72
<210> 20
   <211> 75
   <212> DNA
   <213> künstliche Sequenz
<220>
   <223> Beschreibung der künstlichen Sequenz: DNA
<400> 20
   aagatcgcca aggtcttcaa gaaccccaag ttcagcattc tgaccgtgaa caatgacatc 60
   accctgctga agctg 75
<210> 21
   <211 > 72
   <212> DNA
   <213> künstliche Sequenz
<220>
   <223> Beschreibung der künstlichen Sequenz: DNA
<400> 21
   gccacacctg cccgcttctc ccagacagtg tccgccgtgt gcctgcccag cgccgacgac 60
   gacttccccg cg 72
<210> 22
   <211> 72
   <212> DNA
   <213> künstliche Sequenz
<220>
   <223> Beschreibung der künstlichen Sequenz: DNA
<400> 22
   gggacactgt gtgccaccac aggctggggc aagaccaagt acaacgccaa caagacccct 60
   gacaagctgc ag 72
<210> 23
   <211> 72
   <212> DNA
   <213> künstliche Sequenz
<220>
   <223> Beschreibung der künstlichen Sequenz: DNA
<40.0> 23
<210> 24
   <211> 69
   <212> DNA
   <213> künstliche Sequenz
<220>
   <223> Beschreibung der künstlichen Sequenz: DNA
<400> 24
   atctgtgccg gggccagtgg cgtctcctcc tgcatgggcg actctggcgg tcccctggtc 60
   tgccaaaag 69
<210> 25
   <211> 72
   <212> DNA
   <213> künstliche Sequenz
<220>
   <223> Beschreibung der künstlichen Sequenz: DNA
<400> 25
   gatggagcct ggaccctggt gggcattgtg tcctggggca gcgacacctg ctccacctcc 60
   agccctggcg tg 72
<210> 26
   <211> 54
   <212> DNA
   <213> künstliche Sequenz
<220>
   <223> Beschreibung der künstlichen Sequenz: DNA
<400> 26
   tacgcccgtg tcaccaagct cataccttgg gtgcagaaga tcctggctgc caac 54
<210> 27
   <211> 30
   <212> DNA
   <213> künstliche Sequenz
<220>
   <223> Beschreibung der künstlichen Sequenz: DNA
<400> 27
   caggccgctg agcacagggt ggatggcggg 30
<210> 28
   <211 > 30
   <212> DNA
   <213> künstliche Sequenz
<220>
   <223> Beschreibung der künstlichen Sequenz: DNA
<400> 28
   gccggttttg tcctgcaggg acacctgcca 30
<210> 29
   <211> 30
   <212> DNA
   <213> künstliche Sequenz
<220>
   <223> Beschreibung der künstlichen Sequenz: DNA
<400> 29
   gtcggaggtg cggaccccgc agtgggcagc 30
<210> 30
   <211 > 30
   <212> DNA
   <213> künstliche Sequenz
<220>
   <223> Beschreibung der künstlichen Sequenz: DNA
<400> 30
   gaccttggcg atcttcagga cctggatgtt 30
<210> 31
   <211> 30
   <212> DNA
   <213> künstliche Sequenz
<220>
   <223> Beschreibung der künstlichen Sequenz: DNA
<400> 31
   gcgggcaggt gtggccagct tcagcagggt 30
<210> 32
   <211> 30
   <212> DNA
   <213> künstliche Sequenz
<220>
   <223> Beschreibung der künstlichen Sequenz: DNA
<400> 32
   ggcacacagt gtccccgcgg ggaagtcgtc 30
<210> 33
   <211> 30
   <212> DNA
   <213> künstliche Sequenz
<220>
   <223> Beschreibung der künstlichen Sequenz: DNA
<400> 33
   gggcagggct gcctgctgca gcttgtcagg 30
<210> 34
   <211> 30
   <212> DNA
   <213> künstliche Sequenz
<220>
   <223> Beschreibung der künstlichen Sequenz: DNA
<400> 34
   ggccccggca cagatcatca cgtcggtgat 30
<210> 35
   <211> 30
   <212> DNA
   <213> künstliche Sequenz
<220>
   <223> Beschreibung der künstlichen Sequenz: DNA
<400> 35
   ggtccaggct ccatcctttt ggcagaccag 30
<210> 36
   <211>30
   <212> DNA
   <213> künstliche Sequenz
<220>
   <223> Beschreibung der künstlichen Sequenz: DNA
<400> 36
   ggtgacacgg gcgtacacgc cagggctgga 30
<210> 37
   <211> 26
   <212> DNA
   <213> künstliche Sequenz
<220>
   <223> Beschreibung der künstlichen Sequenz: DNA
<400> 37
   gggaattcca tatggctttc ctctgg 26
<210> 38
   <211> 27
   <212> DNA
   <213> künstliche Sequenz
<220>
   <223> Beschreibung der künstlichen Sequenz: DNA
<400> 38
   ccgctcgagt tggcagccag gatcttc 27
<210> 39
   <211> 643
   <212> DNA
   <213> künstliche Sequenz
<220>
   <223> Beschreibung der künstlichen Sequenz: DNA
<400> 39
   tttccctcta gaaataattt tgtttaactt taagaaggag atatcatatg agtgctggta 60
   ttaactacgt gcaaaactac aacggcaacc ttgctgattt cacctatgac gagagtgccg 120
   gaacattttc catgtactgg gaagatggag tgagctccga ctttgtcgtt ggtctgggct 180
   ggaccactgg ttcttcgaat gctatcagct actctgccga atacagtgct tctggctcct 240
   cttcctacct cgctgtgtac ggctgggtta actatcctca ggctgaatac tacatcgtcg 300
   aggattacgg tgattacaac ccttgcagct cggccacaag ccttggtacc gtgtactctg 360
   atggaagcac ctaccaagtc tgcaccgaca ctcgaactaa cgaaccatcg atcacgggaa 420
   caagcacgtt cacgcagtac ttctccgttc gagagagcac gcgcacatct ggaacggtga 480
   ctgttgccaa ccatttcaac ttctgggccc agcatgggtt cgggaattcc gacttcaatt 540
   atcaggtcat ggcagtggaa gcatggagcg gcgccggcag cgccagtgtc acgatctcct 600
   ctaaactcga gcaccaccac caccaccact gagatccggc tgc 643
<210> 40
   <211> 643
   <212> DNA
   <213> künstliche Sequenz
<220>
   <223> Beschreibung der künstlichen Sequenz: DNA
<400> 40
   aaagggagat ctttattaaa acaaattgaa attcttcctc tatagtatac tcacgaccat 60
   aattgatgca cgttttgatg ttgccgttgg aacgactaaa gtggatactg ctctcacggc 120
   cttgtaaaag gtacatgacc cttctacctc actcgaggct gaaacagcaa ccagacccga 180
   cctggtgacc aagaagctta cgatagtcga tgagacggct tatgtcacga agaccgagga 240
   gaaggatgga gcgacacatg ccgacccaat tgataggagt ccgacttatg atgtagcagc 300
   tcctaatgcc actaatgttg ggaacgtcga gccggtgttc ggaaccatgg cacatgagac 360
   taccttcgtg gatggttcag acgtggctgt gagcttgatt gcttggtagc tagtgccctt 420
   gttcgtgcaa gtgcgtcatg aagaggcaag ctctctcgtg cgcgtgtaga ccttgccact 480
   gacaacggtt ggtaaagttg aagacccggg tcgtacccaa gcccttaagg ctgaagttaa 540
   tagtccagta ccgtcacctt cgtacctcgc cgcggccgtc gcggtcacag tgctagagga 600
   gatttgagct cgtggtggtg gtggtggtga ctctaggccg acg 643
<210> 41
   <211> 861
   <212> DNA
   <213> künstliche Sequenz
<220>
   <223> Beschreibung der künstlichen Sequenz: DNA
<400> 41
   ttaactttaa gaaggagata tacatatggc tttcctctgg ctcctctcct gctgggccct 60
   cctgggtacc accttcggct gcggggtccc cgccatccac cctgtgctca gcggcctgtc 120
   ccgcatcgtg aatggggagg acgccgtccc cggctcctgg ccctggcagg tgtccctgca 180
   ggacaaaacc ggcttccact tctgcggggg ctccctcatc agcgaggact gggtggtcac 240
   cgctgcccac tgcggggtcc gcacctccga cgtggtcgta gctggtgagt ttgatcaagg 300
   ctctgacgag gagaacatcc aggtcctgaa gatcgccaag gtcttcaaga accccaagtt 360
   cagcattctg accgtgaaca atgacatcac cctgctgaag ctggccacac ctgcccgctt 420
   ctcccagaca gtgtccgccg tgtgcctgcc cagcgccgac gacgacttcc ccgcggggac 480
   actgtgtgcc accacaggct ggggcaagac caagtacaac gccaacaaga cccctgacaa 540
   gctgcagcag gcagccctgc ccctcctgtc caatgccgaa tgcaagaagt cctggggccg 600
   ccgcatcacc gacgtgatga tctgtgccgg ggccagtggc gtctcctcct gcatgggcga 660
   ctctggcggt cccctggtct gccaaaagga tggagcctgg accctggtgg gcattgtgtc 720
   ctggggcagc gacacctgct ccacctccag ccctggcgtg tacgcccgtg tcaccaagct 780
   cataccttgg gtgcagaaga tcctggctgc caacctcgag caccaccacc accaccactg 840
   agatccggct gctaacaaag c 861
<210> 42
   <211> 861
   <212> DNA
   <213> künstliche Sequenz
<220>
   <223> Beschreibung der künstlichen Sequenz: DNA
<400> 42
   aattgaaatt cttcctctat atgtataccg aaaggagacc gaggagagga cgacccggga   60
   ggacccatgg tggaagccga cgccccaggg gcggtaggtg ggacacgagt cgccggacag  120
   ggcgtagcac ttacccctcc tgcggcaggg gccgaggacc gggaccgtcc acagggacgt  180
   cctgttttgg ccgaaggtga agacgccccc gagggagtag tcgctcctga cccaccagtg  240
   gcgacgggtg acgccccagg cgtggaggct gcaccagcat cgaccactca aactagttcc  300
   gagactgctc ctcttgtagg tccaggactt ctagcggttc cagaagttct tggggttcaa  360
   gtcgtaagac tggcacttgt tactgtagtg ggacgacttc gaccggtgtg gacgggcgaa  420
   gagggtctgt cacaggcggc acacggacgg gtcgcggctg ctgctgaagg ggcgcccctg  480
   tgacacacgg tggtgtccga ccccgttctg gttcatgttg cggttgttct ggggactgtt  540
   cgacgtcgtc cgtcgggacg gggaggacag gttacggctt acgttcttca ggaccccggc  600
   ggcgtagtgg ctgcactact agacacggcc ccggtcaccg cagaggagga cgtacccgct  660
   gagaccgcca ggggaccaga cggttttcct acctcggacc tgggaccacc cgtaacacag  720
   gaccccgtcg ctgtggacga ggtggaggtc gggaccgcac atgcgggcac agtggttcga  780
   gtatggaacc cacgtcttct aggaccgacg gttggagctc gtggtggtgg tggtggtgac  840
   tctaggccga cgattgtttc g                                            861